(19) Europäisches Patentamt / European Patent Office / Office européen des brevets

(11) EP 4 570 787 A1

(12) **EUROPEAN PATENT APPLICATION**
published in accordance with Art. 153(4) EPC

(43) Date of publication:
**18.06.2025 Bulletin 2025/25**

(21) Application number: **23852103.3**

(22) Date of filing: **11.08.2023**

(51) International Patent Classification (IPC):
***C07D 209/86*** (2006.01) ***C07D 307/91*** (2006.01) ***C09K 11/06*** (2006.01) ***H05B 33/14*** (2006.01) ***H10K 50/10*** (2023.01)

(52) Cooperative Patent Classification (CPC):
**C07D 209/86; C07D 307/91; C09K 11/06; H05B 33/14; H10K 50/10**

(86) International application number:
**PCT/IB2023/058126**

(87) International publication number:
**WO 2024/033884 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.08.2022 KR 20220101083**

(71) Applicant: **Hodogaya Chemical Co., Ltd.**
**Tokyo, 105-0021 (JP)**

(72) Inventors:
- **KASE, Kouki**
  **Tokyo 105-0021 (JP)**
- **KO, Sang-Won**
  **Tokyo 105-0021 (JP)**
- **LIM, Jae-Geon**
  **Tokyo 105-0021 (JP)**
- **IZUMIDA, Junichi**
  **Tokyo 105-0021 (JP)**
- **HAYASHI, Shuichi**
  **Tokyo 105-0021 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) ARYLAMINE COMPOUND, ORGANIC ELECTROLUMINESCENCE ELEMENT, AND ELECTRONIC DEVICE

(57) The present disclosure aims to provide an organic compound represented by the following general formula (I) that serves as a material for high-efficiency and durable organic EL devices, offering superior characteristics such as excellent hole injection and transport performance, electron-blocking capability, and high stability in thin-film states. Additionally, the present disclosure is to provide a high-efficiency, durable organic EL device utilizing the compound.

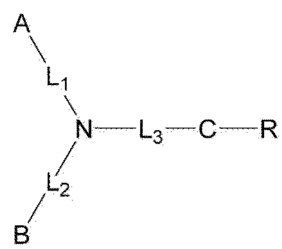

( I )

wherein:
A represents a substituted or unsubstituted aromatic hydrocarbon group, a substituted or
unsubstituted aromatic heterocyclic group, or a substituted or unsubstituted fused polycyclic aromatic group;
B represents a substituted or unsubstituted carbazolyl group;
C represents an unsubstituted naphthylene group;
R represents an unsubstituted aromatic hydrocarbon

EP 4 570 787 A1

group, an unsubstituted aromatic heterocyclic group, or an unsubstituted fused polycyclic aromatic group;

$L_1$ to $L_3$ represent a single bond, an unsubstituted divalent aromatic hydrocarbon group, an unsubstituted divalent aromatic heterocyclic group, or an unsubstituted divalent fused polycyclic aromatic group.

The arylamine compounds of the present disclosure exhibit excellent thermal stability and superior hole transport properties. Organic EL devices incorporating these compounds into their hole transport layer, electron-blocking layer, emission layer, or hole injection layer demonstrated favorable device characteristics.

## Description

TECHNICAL FIELD

The present disclosure relates to compounds and devices suitable for organic electroluminescence devices (hereinafter abbreviated as "organic EL devices"), which are self-luminous devices ideal for various display applications. Specifically, the present disclosure pertains to arylamine compounds and organic EL devices employing such compounds.

BACKGROUND

Organic EL devices, being self-luminous, are brighter and exhibit superior visibility compared to liquid crystal devices, enabling vivid displays. Thus, extensive research has been conducted in this field.
In 1987, C.W. Tang et al. of Eastman Kodak developed a layered structure device, assigning various roles to specific materials, thereby making organic EL devices practical. By layering an electron-transporting phosphor and a hole-transporting organic material, and injecting both charges into the phosphor layer to induce light emission, high brightness exceeding 1000 cd/$m^2$ at voltages below 10V can be achieved (e.g., see Patent Literatures 1 and 2).
Since then, significant improvements have been made to commercialize organic EL devices. By further segmenting the roles of layered structures, field-emission devices have been developed that sequentially stack an anode, a hole injection layer, a hole transport layer, an emission layer, an electron transport layer, an electron injection layer, and a cathode on a substrate. This configuration has enabled high efficiency and durability (e.g., see Non-Patent Literature 1).
Additionally, to further enhance luminous efficiency, attempts have been made to utilize triplet excitons, and the use of phosphorescent compounds has been explored (e.g., see Non-Patent Literature 2). Furthermore, devices employing light emission through thermally activated delayed fluorescence (TADF) have been developed. In 2011, Adachi et al. from Kyushu University achieved an external quantum efficiency of 5.3% with a device utilizing a TADF material (e.g., see Non-Patent Literature 3).
The emission layer is typically fabricated by doping a fluorescent compound, a phosphorescent compound, or a material that emits delayed fluorescence into a charge-transporting compound, commonly referred to as a host material. As described in the aforementioned non-patent literature, the choice of organic materials in an organic EL device significantly impacts its properties, such as its efficiency or durability (e.g., see Non-Patent Literature 2).
In organic EL devices, light emission occurs as charges injected from both electrodes recombine in the emission layer. It is critical how to achieve efficient delivery of holes and electrons to the emission layer. Therefore, it is necessary to design devices with excellent carrier balance. By using materials with high hole-injecting properties to supply holes to the emission layer from the anode, and materials with high electron-blocking properties to block electrons injected from the cathode, the recombination probability of holes and electrons within the emission layer is enhanced. Furthermore, by trapping excitons generated in the emission layer, high luminous efficiency can be achieved. To this end, the role of hole-transporting materials is crucial. These materials must possess high hole-injecting properties, high hole mobility, strong electron-blocking properties, and excellent durability against electrons.
Additionally, the thermal stability and amorphousness of materials are essential for the longevity of devices. Materials with low thermal stability degrade due to thermal decomposition even at low temperatures during device operation. In materials with low amorphousness, devices degrade due to crystallizing in thin films even a short period. Therefore, materials used in such devices must exhibit high thermal stability and excellent amorphous properties.
Thus far, hole-transporting materials used in organic EL devices include N,N'-diphenyl-N,N'-di($\alpha$-naphthyl) benzidine (NPD) and various aromatic amine derivatives (e.g., see Patent Literatures 1 and 2). While NPD demonstrates favorable hole-transporting capabilities, its glass transition temperature (Tg), which indicates thermal stability, is as low as 96°C. This results in crystallization under high-temperature conditions, leading to degradation of device performance (e.g., see Non-Patent Literature 4).
Moreover, among the aromatic amine derivatives described in the aforementioned patent literature, some compounds exhibit excellent hole mobility of $10^{-3}$ $cm^2$/Vs or higher (e.g., see Patent Literatures 1 and 2). However, their electron-blocking properties are insufficient, allowing some electrons to escape from the emission layer, thereby limiting the potential for improving luminous efficiency. To achieve further improvements in device efficiency, materials with superior electron-blocking properties, enhanced thin-film stability, and high thermal stability are required. Reports exist on highly durable aromatic amine derivatives (e.g., see Patent Literature 3), but these have been used as charge-transporting materials in electrophotographic photoreceptors, not in organic EL devices.
To address these challenges, compounds with improved thermal stability and hole-injecting properties, such as substituted carbazole structures and arylamine compounds, have been proposed (e.g., see Patent Literatures 4 and 5). While devices using these compounds in the hole-injection or hole-transporting layers have shown improvements in

device lifespan and luminous efficiency, these improvements remain insufficient. There is still a demand for devices with lower driving voltage, higher luminous efficiency, and extended lifespan.

[Prior Art Documents]

[Patent Literature]

(Patent Literature 1) US Patent No. 5,792,557
(Patent Literature 2) US Patent No. 5,639,914
(Patent Literature 3) US Patent No. 7,759,030
(Patent Literature 4) JP2009-076817A1
(Patent Literature 5) JP6674892B2
(Patent Literature 6) EP JP Patent No. 2,684,932
(Patent Literature 7) KR Patent No. 101888249B1
(Patent Literature 8) KR Patent No. 102259465B1
(Patent Literature 9) KR Patent No. 102078171B1

[Non-Patent Literature]

(Non-Patent Literature 1) Proceedings of the ninth Applied Physics Lecture Series, pp. 55-61 (2001)
(Non-Patent Literature 2) Proceedings of the ninth Applied Physics Lecture Series, pp. 23-31 (2001)
(Non-Patent Literature 3) Appl. Phys. Let., 98, 083302 (2011)
(Non-Patent Literature 4) OLED Forum, proceedings of the third meeting, pp. 13-14 (2006)

DISCLOSURE

TECHNICAL PROBLEM

The present disclosure aims to develop highly efficient and durable organic EL devices by providing materials for organic EL devices that exhibit: (1) excellent hole injection and transport performances, (2) electron-blocking capability, (3) high stability in thin-film states, and (4) superior durability.

By utilizing the materials of the present disclosure, it is possible to achieve organic EL devices with the following characteristics: (1) high luminous efficiency and power efficiency, (2) low light-emission initiation voltage and practical driving voltage, and (3) extended lifespan.

TECHNICAL SOLUTION

To achieve the goals, the inventors focused on arylamine compounds, which are notable for their excellent hole injection and transport properties, thin-film stability, and durability. By introducing a carbazolyl group to widen the bandgap, optimizing the introduction and substitution positions of substituted naphthylenes, and pursuing material property enhancements, significant improvements were achieved. The resulting organic EL devices demonstrated improved luminous efficiency and power efficiency, reduced emission initiation voltage and practical driving voltage, and a lifespan surpassing conventional devices, leading to the completion of the present disclosure.

1) Specifically, the present disclosure provides an arylamine compound represented by the following General Formula (I):

General Formula (I):

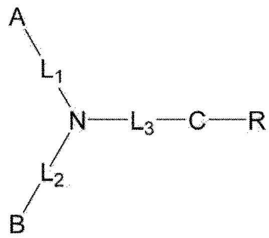

( I )

A represents a substituted or unsubstituted aromatic hydrocarbon group, a substituted or unsubstituted aromatic heterocyclic group, or a substituted or unsubstituted fused polycyclic aromatic group,
B represents a substituted or unsubstituted carbazolyl group,
C represents an unsubstituted naphthylene group,
R represents an unsubstituted aromatic hydrocarbon group, an unsubstituted aromatic heterocyclic group, or an unsubstituted fused polycyclic aromatic group, and
$L_1$ to $L_3$ represent a single bond, an unsubstituted divalent aromatic hydrocarbon group, an unsubstituted divalent aromatic heterocyclic group, or an unsubstituted divalent fused polycyclic aromatic group.

2) The present disclosure further specifies the arylamine compound of the General Formula (I) as set forth in 1), wherein C is an unsubstituted 1,2-naphthylene group, an unsubstituted 1,3-naphthylene group, an unsubstituted 2,4-naphthylene group, an unsubstituted 2,5-naphthylene group, an unsubstituted 2,6-naphthylene group, an unsubstituted 2,7-naphthylene group, or an unsubstituted 2,8-naphthylene group.
3) The present disclosure further specifies the arylamine compound of the General Formula (I) as set forth in 2), wherein $L_2$ and $L_3$ are an unsubstituted phenylene group or an unsubstituted biphenylene group.
4) The present disclosure further specifies the arylamine compound of the General Formula (I) as set forth in 3), wherein R is an unsubstituted phenyl group, an unsubstituted naphthyl group, an unsubstituted dibenzofuranyl group, an unsubstituted phenanthrenyl group, or an unsubstituted biphenyl group.
5) The present disclosure further specifies the arylamine compound of the General Formula (I) as set forth in 4), wherein B is a substituted or unsubstituted 9-carbazolyl group, a substituted or unsubstituted 2-carbazolyl group, or a substituted or unsubstituted 3-carbazolyl group.
6) The present disclosure further specifies the arylamine compound of the General Formula (I) as set forth in 5), wherein B is an unsubstituted 9-carbazolyl group.
7) The present disclosure provides an organic EL device comprising a pair of electrodes and at least one organic layer interposed therebetween, wherein the organic layer comprises the arylamine compound set forth in 1) and 2).
8) The present disclosure further specifies the organic EL device as set forth in 7) wherein the organic layer is a hole transport layer.
9) The present disclosure further specifies the organic EL device as set forth in 7), wherein the organic layer is an electron-blocking layer.
10) The present disclosure further specifies the organic EL device as set forth in 7), wherein the organic layer is a hole injection layer.
11) The present disclosure further specifies the organic EL device as set forth in 7), wherein the organic layer is an emission layer.
12) The present disclosure also provides an electronic device including a pair of electrodes and at least one organic layer interposed therebetween, wherein the organic layer includes the arylamine compound set forth in 1) and 2).

In General Formula (I), the "substituted or unsubstituted aromatic hydrocarbon group," "substituted or unsubstituted aromatic heterocyclic group," or "substituted or unsubstituted fused polycyclic aromatic group" represented by A specifically includes phenyl, biphenyl, terphenyl, naphthyl, anthracenyl, phenanthrenyl, indenyl, pyrenyl, perylenyl, fluoranthenyl, triphenylenyl, fluorenyl, spirobifluorenyl, pyridyl, pyrimidinyl, triazinyl, furyl, pyrrolyl, thienyl, quinolyl, isoquinolyl, benzofuranyl, benzothienyl, indolyl, carbazolyl, benzoxazolyl, benzothiazolyl, azafluorenyl, diazafluorenyl, azaspirobifluorenyl, diazaspirobifluorenyl, quinoxalinyl, benzimidazolyl, pyrazolyl, dibenzofuranyl, dibenzothienyl, naphthyridinyl, phenanthrolinyl, acridinyl, and carbazolinyl. Other examples include an aryl of 6 to 30 carbon atoms and a heteroaryl of 2 to 20 carbon atoms.
In the "substituted aromatic hydrocarbon group," "substituted aromatic heterocyclic group," or "substituted fused polycyclic aromatic group" represented by A of General Formula (I), the substituent specifically includes: a deuterium atom, a cyano, and a nitro; a halogen atom such as fluorine, chlorine, bromine, or iodine; a silyl such as trimethylsilyl or

triphenylsilyl; a straight or branched alkyl of 1 to 6 carbon atoms, such as methyl, ethyl, propyl, etc.; a straight or branched alkoxy of 1 to 6 carbon atoms, such as methoxy, ethoxy, propoxy, etc.; an alkenyl such as vinyl, allyl, etc.; an aryloxy such as phenyloxy, tolyloxy, etc.; arylalkyloxy such as benzyloxy, phenetyloxy, etc.; an aromatic hydrocarbon or fused polycyclic aromatic hydrocarbon such as phenyl, biphenyl, terphenyl, naphthyl, anthracenyl, phenanthrenyl, fluorenyl, spirobifluorenyl, indenyl, pyrenyl, perylenyl, fluoranthenyl, triphenylenyl, etc.; an aromatic heterocyclic ring such as pyridyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzimidazolyl, pyrazolyl, dibenzofuranyl, dibenzothienyl, carbazolinyl, etc. These substituents may themselves be substituted with other substituents from the examples above. Additionally, two substituents on the same benzene ring or different benzene rings, may be linked through a single bond, substituted or unsubstituted methylene group, oxygen atom, or sulfur atom to form a ring structure.

In the "substituted or unsubstituted carbazolyl group represented by B in General Formula (I), the substituents are the same as those in the "substituted aromatic hydrocarbon group," "substituted aromatic heterocyclic group," or "substituted fused polyaromatic group" represented by A in General Formula (I), and possible embodiments may also be the same embodiments as the exemplified embodiments.

The "aromatic hydrocarbon group," "aromatic heterocyclic group," or "fused polyaromatic group" represented by R in General Formula (I) may be same as the "aromatic hydrocarbon group," "aromatic heterocyclic group," or "fused polyaromatic group" in the "substituted or unsubstituted aromatic hydrocarbon group," "substituted or unsubstituted heteroaromatic group," or "substituted or unsubstituted fused polyaromatic group" represented by A in General Formula (I), and possible embodiments may also be the same embodiments as the exemplified embodiments.

The "divalent aromatic hydrocarbon group," "divalent aromatic heterocyclic group," or "divalent fused polycyclic aromatic group," represented by Li to $L_3$ in General Formula (I) may be the same as the "aromatic hydrocarbon group," "aromatic heterocyclic group," or "fused polyaromatic group" in the "substituted or unsubstituted aromatic hydrocarbon group," "substituted or unsubstituted aromatic heterocyclic group," or "substituted or unsubstituted fused polyaromatic group" represented by A in General Formula (I), with one hydrogen atom removed therefrom.

$L_2$ in General Formula (I) is preferably a biphenylene group and more preferably a 2,4'-biphenylene or 3,4'-biphenylene group.

$L_3$ in General Formula (I) is preferably a phenylene group and more preferably a 1,4-phenylene group.

R in General Formula (I) is more preferably a phenyl, naphthyl group.

C in General Formula (I) is more preferably a 1,3-naphthylene, 2,4-naphthylene, 2,5-naphthylene, or 2,8-naphthylene group.

The arylamine compounds represented by General Formula (I), suitable as materials for organic EL devices, according to the present disclosure, are preferably used as components for the hole injection layer, hole transport layer, electron blocking layer, or emission layer, with more preference for use in the hole transport layer or electron-blocking layer.

## ADVANTAGEOUS EFFECTS

The arylamine compounds of the present disclosure exhibit the following advantages over conventional hole transport materials: (1) excellent hole injection properties, (2) high hole mobility, (3) superior electron-blocking capability, (4) high resistance to electrons, (5) stability in thin-film states, and (6) outstanding thermal stability. By incorporating the arylamine compounds of the present disclosure into organic EL devices, the following properties are achieved: (7) high luminous efficiency, (8) low light emission onset voltage, (9) low practical operating voltage, and (10) long lifespan.

In particular, the arylamine compounds of the present disclosure feature excellent electron-blocking capabilities and high electron resistance, while also being stable in thin-film states, effectively trapping excitons generated within the emission layer. As a result, organic EL devices having these compounds as electron-blocking materials demonstrate improved recombination probabilities for holes and electrons, suppressed thermal deactivation, and increased luminous efficiency. Furthermore, reduced driving voltage enhances current resistance, leading to improved maximum luminous brightness.

## DESCRIPTION OF DRAWINGS

FIG. 1 is a view showing preferred examples of arylamine compounds represented by General Formula (I), specifically compounds (1) to (12).

FIG. 2 is a view showing preferred examples of arylamine compounds represented by General Formula (I), specifically compounds (13) to (24).

FIG. 3 is a view showing preferred examples of arylamine compounds represented by General Formula (I), specifically

compounds (25) to (36).

FIG. 4 is a view showing preferred examples of arylamine compounds represented by General Formula (I), specifically compounds (37) to (48).

FIG. 5 is a view showing preferred examples of arylamine compounds represented by General Formula (I), specifically compounds (49) to (60).

FIG. 6 is a view showing preferred examples of arylamine compounds represented by General Formula (I), specifically compounds (61) to (72).

FIG. 7 is a view showing preferred examples of arylamine compounds represented by General Formula (I), specifically compounds (73) to (84).

FIG. 8 is a view showing preferred examples of arylamine compounds represented by General Formula (I), specifically compounds (85) to (96).

FIG. 9 is a view showing preferred examples of arylamine compounds represented by General Formula (I), specifically compounds (97) to (108).

FIG. 10 is a view showing preferred examples of arylamine compounds represented by General Formula (I), specifically compounds (109) to (120).

FIG. 11 is a view showing preferred examples of arylamine compounds represented by General Formula (I), specifically compounds (121) to (135).

FIG. 12 is a view showing preferred examples of arylamine compounds represented by General Formula (I), specifically compounds (136) to (147).

FIG. 13 is a view showing preferred examples of arylamine compounds represented by General Formula (I), specifically compounds (148) to (161).

FIG. 14 is a view showing preferred examples of arylamine compounds represented by General Formula (I), specifically compounds (162) to (176).

FIG. 15 is a view showing preferred examples of arylamine compounds represented by General Formula (I), specifically compounds (177) to (188).

FIG. 16 is a view showing preferred examples of arylamine compounds represented by General Formula (I), specifically compounds (189) to (203).

FIG. 17 is a view showing preferred examples of arylamine compounds represented by General Formula (I), specifically compounds (204) to (215).

FIG. 18 is a view showing preferred examples of arylamine compounds represented by General Formula (I), specifically compounds (216) to (228).

FIG. 19 is a view showing preferred examples of arylamine compounds represented by General Formula (I), specifically compounds (229) to (239).

FIG. 20 is a view showing preferred examples of arylamine compounds represented by General Formula (I), specifically compounds (240) to (242).

FIG. 21 is a view showing preferred examples of arylamine compounds represented by General Formula (I), specifically compounds (253) to (261).

FIG. 22 is a schematic diagram showing the structure of organic EL devices in Examples 21 to 38 and Comparative Examples 1 to 3.

## MODE FOR INVENTION

The arylamine compounds of the present disclosure, though novel, can be synthesized following well-established methods.

Preferred examples of the arylamine compounds represented by General Formula (I), suitable for use in the organic EL devices of the present disclosure, are depicted in FIGS. 1 to 21, but are not limited thereto.

Purification of the arylamine compounds represented by General Formula (I) can be conducted using conventional techniques, such as column chromatography, adsorption methods utilizing silica gel, activated carbon, or activated clay, recrystallization or precipitation with solvents, sublimation techniques, etc. Identification of these compounds can be performed via NMR analysis. Relevant physical properties include melting point, glass transition temperature (Tg), and work function. Melting points indicate suitability for vapor deposition, glass transition temperatures (Tg) reflect thin-film stability, and work functions provide insights into hole injection, hole transport properties, and electron-blocking capability.

Melting point and glass transition temperature (Tg) can be measured using differential scanning calorimetry (e.g., Bruker AXS DSC3100SA) on powdered samples.

The work function can be determined by creating a 100 nm thin film on an ITO substrate and measuring with an ionization potential measurement device (e.g., PYS-202, Sumitomo Heavy Industries Ltd.).

The structure of the organic EL device of the present disclosure may be configured to include an anode, a hole injection layer, a hole transport layer, an emission layer, an electron transport layer, an electron injection layer, and a

cathode sequentially provided on a substrate, with optional arrangement of an electron blocking layer between the hole transport layer and the emission layer and a hole blocking layer between the emission layer and the electron transport layer. In multi-layer configurations, a single organic layer may perform multiple functions, e.g., serving as both a hole injection and hole transport layer or as both an electron injection and transport layer. Layers with identical functions can be stacked in multiple layers, such as dual-stacked hole transport layers, emission layers, and electron transport layers.

The anode material for the organic EL device of the present disclosure includes electrode materials with a high work function, such as ITO or gold. For the hole injection layer material in the organic EL device of the present disclosure, materials such as porphyrin compounds exemplified by copper phthalocyanine, starburst-type triphenylamine derivatives, arylamine compounds that possess two or more triphenylamine moieties or carbazolyl moieties within the molecule where each moiety is connected via a single bond or a divalent group that does not include heteroatoms, acceptor-type heterocyclic compounds such as hexacyanoazatriphenylene, and coating-type polymeric materials, can be used. These materials may form thin films through known methods such as vapor deposition, spin coating, or inkjet printing.

The arylamine compounds of the present disclosure exhibit excellent hole injection and transport performance, stability in thin-film states, and durability. Consequently, organic EL devices incorporating these compounds as materials for the hole injection and/or transport layer achieve improved hole transport efficiency in the emission layer, enhanced luminous efficiency, and reduced driving voltage, thereby improving the durability of the device. This results in the realization of high-efficiency, low driving voltage, and long-lifetime characteristics.

In addition to the arylamine compounds of the present disclosure, materials such as N,N'-diphenyl-N,N'-di(m-tolyl)-benzidine (TPD), N,N'-diphenyl-N,N'-di($\alpha$-naphthyl)-benzidine (NPD), and N,N,N',N'-tetraphenylbenzidine, benzidine derivatives, 1,1-bis[(di-4-tolylamino)phenyl]cyclohexane (TAPC), and arylamine compounds possessing two or more triphenylamine or carbazolyl structures within the molecule, each connected by a single bond or a divalent group free from heteroatoms, can also be used as materials for the hole injection layer and hole transport layer in the organic EL devices of the present disclosure. These materials can be used to form thin films individually, as a mixture of multiple materials, or in single layers. Moreover, they can be arranged in stacked structures consisting of individual layers of the materials, layers formed by mixing multiple materials, or combinations of single-material layers and mixed-material layers. Additionally, polymeric materials suitable for coating, such as poly(3,4-ethylenedioxythiophene)/poly(styrene sulfonate) (PEDOT/PSS), can be used as materials for the hole injection and transport layers. Thin films of these materials can be formed using known methods such as vapor deposition, spin coating, or inkjet printing.

Furthermore, the hole injection or transport layers can incorporate materials typically used for such layers, doped with tris(bromophenylamino)hexachloroantimonate or radialene derivatives (e.g., as described in Patent Literature 6). Polymeric compounds containing structures derived from benzidine derivatives such as TPD can also be employed.

The arylamine compounds of the present disclosure are characterized by excellent electron blocking ability, high electron resistance, and stability in thin-film states, allowing them to trap excitons generated in the emission layer. By using these compounds as electron blocking materials to fabricate electron blocking layers in organic EL devices, the recombination probability of holes and electrons is enhanced, suppressing thermal quenching. As a result, devices achieve high luminous efficiency, reduced driving voltage, and improved current resistance, leading to enhanced maximum brightness.

As materials for the electron blocking layer in the organic EL device of the present disclosure, in addition to the arylamine compounds of the present disclosure, compounds with electron-blocking properties can be used, for example, carbazole derivatives such as 4,4',4"-tri(N-carbazolyl)triphenylamine (TCTA), 9,9-bis[4-(carbazol-9-yl)phenyl]fluorene, 1,3-bis(carbazol-9-yl)benzene (mCP), 2,2-bis(4-carbazol-9-ylphenyl)adamantane (Ad-Cz), and compounds having triphenylsilyl groups and triarylamine structures, exemplified by 9-[4-(carbazol-9-yl)phenyl]-9-[4-(triphenylsilyl)phenyl]-9H-fluorene. These materials can also serve as materials for the hole transport layer. These materials can be used to form thin films individually, as mixtures of multiple materials, or as single layers. Additionally, they can be structured as stacked layers of individually formed thin films, layers formed by mixing multiple materials, or combinations of single-material layers and mixed-material layers. Thin films of these materials can be formed using known methods such as vapor deposition, spin coating, or inkjet printing.

The arylamine compounds of the present disclosure exhibit excellent hole transport properties and a wide band gap. As a result, when these compounds are used as host materials to fabricate the emission layer in an organic EL device, the emission layer, incorporating dopants such as fluorescent emitters, phosphorescent emitters, or delayed fluorescence emitters, demonstrates reduced driving voltage and improved luminous efficiency.

As materials for the emission layer in the organic EL device of the present disclosure, in addition to the arylamine compounds of the present disclosure, other compounds such as tris(8-hydroxyquinolinolato)aluminum ($Alq_3$), metal complexes derived from quinolinol, various metal complexes, anthracene derivatives, bisstyrylbenzene derivatives, pyrene derivatives, oxazole derivatives, and polyparaphenylenevinylene derivatives can be used. In addition, the emission layer may be composed of host materials and dopant materials. As host materials, anthracene derivatives are preferably used. In addition to the arylamine compounds of the present disclosure, other emission materials may include heterocyclic compounds having indole rings or carbazole rings as part of their fused ring structures, carbazole

derivatives, thiazole derivatives, benzimidazole derivatives, and polydialkylfluorene derivatives. As dopant materials, compounds such as quinacridone, coumarin, rubrene, perylene, and their derivatives, as well as benzopyran derivatives, rhodamine derivatives, and aminostyryl derivatives, may be employed. These materials can be formed into thin films either individually or as mixtures of multiple components. They may also be used as single-layer films or in stacked structures comprising layers formed individually, mixed, or in combinations of both. Thin films of these materials may be formed using known methods such as vapor deposition, spin coating, or inkjet printing.

Additionally, it is possible to use phosphorescent emitters as the emission materials. Phosphorescent emitters may include metal complexes of iridium or platinum. Examples include green phosphorescent emitters such as $Ir(ppy)_3$, blue phosphorescent emitters such as FIrpic and FIr6, and red phosphorescent emitters such as $Btp_2Ir(acac)$. For such cases, host materials may include materials with excellent hole injection and transport properties, such as 4,4'-di(N-carbazolyl)biphenyl (CBP), TCTA, mCP, and carbazole derivatives, as well as the arylamine compounds of the present disclosure. Host materials with electron transport properties may include compounds such as p-bis(triphenylsilyl)benzene (UGH2) and 2,2',2"-(1,3,5-phenylene)-tris(1-phenyl-1H-benzimidazole) (TPBI). Using these materials enables the fabrication of high-performance organic EL devices.

For doping phosphorescent emission materials into host materials, it is preferable to perform co-deposition in a range of 1 to 30 weight percent relative to the entire emission layer to avoid concentration quenching.

Additionally, emission materials that emit delayed fluorescence, such as CDCB derivatives including PIC-TRZ, CC2TA, PXZ-TRZ, and 4CzIPN, can also be used (see, for example, Non-Patent Literature 3). These materials can form thin films using known methods such as vapor deposition, spin coating, or inkjet printing.

As materials for the hole-blocking layer in the organic EL device of the present disclosure, compounds with hole-blocking functionality can be used, including phenanthroline derivatives such as bathocuproine (BCP), metal complexes derived from quinolinol derivatives such as bis(2-methyl-8-quinolinolato)-4-(phenylphenolato)aluminum (BAlq), various rare-earth complexes, oxazole derivatives, triazole derivatives, and triazine derivatives. These materials can also serve as electron transport layer materials. Thin films can be formed from these materials either individually or in mixture. They can be used as single-layer films or in stacked structures comprising individually formed layers, mixed layers, or combinations thereof. Thin films of these materials can be formed using known methods such as vapor deposition, spin coating, or inkjet printing.

As materials for the electron transport layer of the organic EL device in the present disclosure, the following can be used: metal complexes derived from quinolinol derivatives such as $Alq_3$ and BAlq, various other metal complexes, triazole derivatives, triazine derivatives, oxadiazole derivatives, pyridine derivatives, pyrimidine derivatives, benzimidazole derivatives, thiadiazole derivatives, anthracene derivatives, carbodiimide derivatives, quinoxaline derivatives, pyridindole derivatives, phenanthroline derivatives, and silole derivatives. These materials can be used individually to form thin films or mixed together to create composite films. They can also be employed in single-layer configurations or in stacked configurations composed of either unmixed layers, mixed layers, or combinations of the two. The thin films of these materials can be created using known methods such as vapor deposition, spin coating, or inkjet printing.

For the electron injection layer of the organic EL device, suitable materials include alkali metal salts such as lithium fluoride and cesium fluoride, alkaline earth metal salts such as magnesium fluoride, metal complexes derived from quinolinol derivatives such as lithium quinolinolate, metal oxides like aluminum oxide, and metals such as ytterbium (Yb), samarium (Sm), calcium (Ca), strontium (Sr), and cesium (Cs). The electron injection layer may be omitted depending on the optimal selection of the electron transport layer and the cathode.

Additionally, for the electron injection and transport layers, metals such as cesium (Cs) may be N-doped into conventional materials for these layers.

For the cathode of the organic EL device of the present disclosure, low work function metals like aluminum, as well as alloys with even lower work functions such as magnesium-silver alloys, magnesium-indium alloys, and aluminum-magnesium alloys, can be employed as electrode materials.

[EXAMPLES]

A better understanding of the present disclosure may be obtained through the following Examples, which are set forth to illustrate, but are not to construed to limit, the present disclosure.

[EXAMPLE 1]

<Synthesis of Compound (2)>

In a nitrogen-purged reaction vessel, a mixture of N-{4-(3-phenylnaphthalen-1-yl)phenyl}-[1,1'-biphenyl]-4-amine ( 9.5g), 9-(4'-chloro-[1,1'-biphenyl]-2-yl)-carbazole (9.0g), t-butoxide sodium (3.1g), bis[tri(t-butylphosphine)] palladium(0) (0.2g), and toluene (95ml) was stirred overnight under reflux while heating. Then, silica gel was added to

the reaction mixture, followed by stirring for 30 minutes. Afterward, the mixture was filtered through Celite at 90°C, and the solvent was removed by vacuum distillation. The resulting crude mixture was separated via column chromatography using silica gel as the stationary phase and a dichloromethane/n-heptane mixture as the eluent. This process afforded Compound (2) as a white powder (6.6 g, yield: 40.6%).

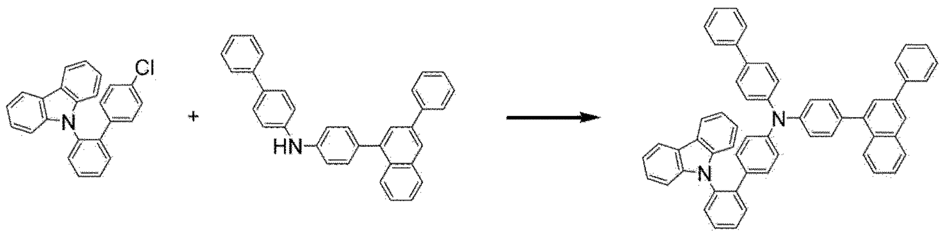

(2)

The structure of the obtained white powder was confirmed using NMR analysis.

The following signals corresponding to 40 protons were detected in the $^{1}H$-NMR($CDCl_3$) spectrum:

δ(ppm)=8.07(2H), 8.05(1H), 7.97(2H), 7.77(2H), 7.73(1H), 7.71(1H), 7.62-7.54(5H), 7.50(4H), 7.42(5H), 7.34(2H), 7.31(3H), 7.23(2H), 7.10(2H), 6.92(4H), 6.82(4H).

[EXAMPLE 2]

<Synthesis of Compound (21)>

In a nitrogen-purged reaction vessel, a mixture of N-phenyl-4-(1-phenylnaphthalen-3-yl)aniline (10.0g), 9-(4'-chloro-[1,1'-biphenyl]-2-yl)-carbazole (10.5g), t-butoxide sodium (3.9g), t-butylphosphine(50wt% toluene solution) (0.4g), tris(dibenzylidene)acetone dipalladium(0) (0.2g), and xylene (100ml) was stirred for 3 hours under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was separated via column chromatography using silica gel as the stationary phase and a dichloromethane/n-heptane mixture as the eluent. This process afforded Compound (21) as a white powder (14.0g, yield: 75.5%).

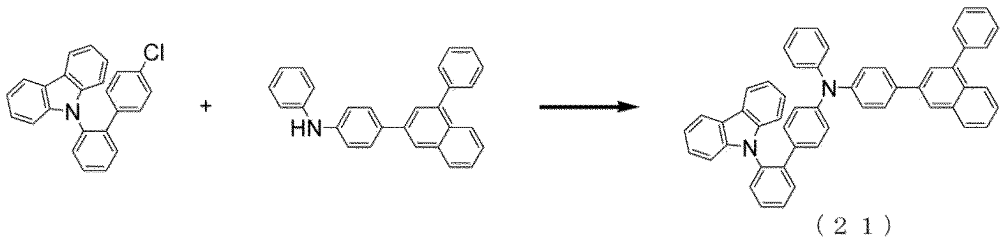

(21)

The structure of the obtained white powder was confirmed using NMR analysis.

The following signals corresponding to 36 protons were detected in the $^{1}H$-NMR($CDCl_3$) spectrum:

δ(ppm)=8.06(2H), 7.99(1H), 7.94(1H), 7.89(1H), 7.71(1H), 7.66(1H), 7.63-7.48(10H), 7.46(1H), 7.40(1H), 7.30(2H), 7.23(2H), 7.16(2H), 7.08(2H), 6.96(1H), 6.85(2H), 6.80(4H), 6.68(2H).

[EXAMPLE 3]

<Synthesis of Compound (22)>

In a nitrogen-purged reaction vessel, a mixture of N- {4-(1-phenylnaphthalen-3-yl)phenyl}-[1,1'-biphenyl]-4-amine (9.5g, 9-(4'-chloro-[1,1'-biphenyl]-2-yl)-carbazole (9.0g), t-butoxide sodium (3.1g), bis[tri(t-butylphosphine)]palladium(0) (0.2g), and toluene (95ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was separated via column chromatography using

silica gel as the stationary phase and a dichloromethane/n-heptane mixture as the eluent. This process afforded Compound (22) as a white powder (6.4g, yield: 39.5 %).

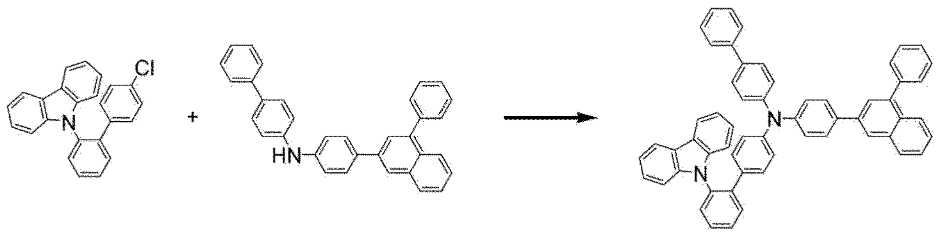

(22)

The structure of the obtained white powder was confirmed using NMR analysis.

The following signals corresponding to 40 protons were detected in the $^1H$-NMR($CDCl_3$) spectrum:

δ(ppm)=8.08(2H), 8.01(1H), 7.95(1H), 7.89(1H), 7.72(1H), 7.67(1H), 7.61(1H), 7.60-7.49(12H), 7.49-7.37(6H), 7.31 (3H), 7.24(1H), 7.09(2H), 6.90(2H), 6.86(2H), 6.83(2H), 6.74(2H).

[EXAMPLE 4]

<Synthesis of Compound (42)>

In a nitrogen-purged reaction vessel, a mixture of N-phenyl-4-(1-phenylnaphthalen-3-yl)aniline (7.0g), 9-(4'-chloro-[1,1'-biphenyl]-3-yl)-carbazole (8.0g), t-butoxide sodium (3.6g), bis[tri(t-butylphosphine)]palladium(0) (0.2g), and toluene (150ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was subjected to purification by precipitation using a dichloromethane/acetone mixture solvent to afforded Compound (42) as a white powder (10.0g, yield: 77.0%).

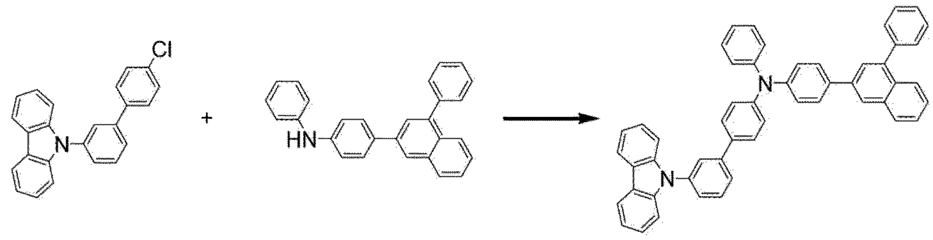

(42)

The structure of the obtained white powder was confirmed using NMR analysis.

The following signals corresponding to 36 protons were detected in the $^1H$-NMR($CDCl_3$) spectrum:

δ(ppm)=8.15(2H), 8.03(1H), 7.93(1H), 7.89(1H), 7.79(1H), 7.69(1H), 7.65(4H), 7.57-7.45(10H), 7.45-7.37(4H), 7.29 (4H), 7.25-7.17(6H), 7.07(1H).

[EXAMPLE 5]

<Synthesis of Compound (43)>

In a nitrogen-purged reaction vessel, a mixture of N- {4-(1-phenylnaphthalen-3-yl)phenyl}-[1,1'-biphenyl]-4-amine (9.0g), 9-(4'-chloro-[1,1'-biphenyl]-3-yl)-carbazole (8.5g), t-butoxide sodium (2.9g), bis[tri(t-butylphosphine)]palladium(0) (0.2g), and toluene (90ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was separated via column chromatography using silica gel as the stationary phase and a dichloromethane/n-heptane mixture as the eluent. This process afforded

Compound (43) as a white powder (3.0g, yield: 19.4%).

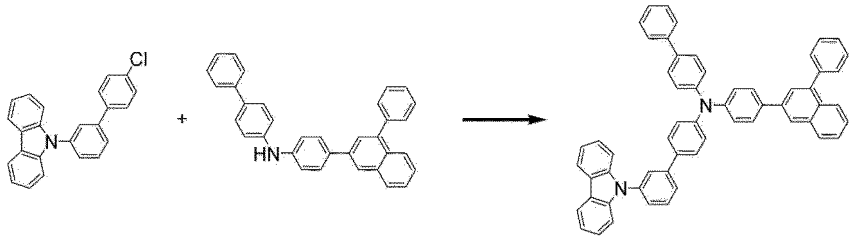

(43)

The structure of the obtained white powder was confirmed using NMR analysis.

The following signals corresponding to 40 protons were detected in the $^1H$-NMR($CDCl_3$) spectrum:

δ(ppm)=8.16(2H), 8.05(1H), 7.95(1H), 7.90(1H), 7.80(1H), 7.69(5H), 7.62-7.47(15H), 7.47-7.38(6H), 7.36-7.24(8H).

[EXAMPLE 6]

<Synthesis of Compound (60)>

In a nitrogen-purged reaction vessel, a mixture of N-([1,1'biphenyl]-4-yl)-4'-(carbazole-9-yl)-[1,1'biphenyl]-4-amine (9.5g), 3-(4-chlorophenyl)-1-phenyl-naphthalene (7.4g), t-butoxide sodium (3.8g), bis[tri(t-butylphosphine)]palladium(0) (0.2g), and toluene (170ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. Purification through recrystallization of the resulting crude mixture in toluene solvent afforded Compound (60) as a white powder (10.9g, yield: 73.0%).

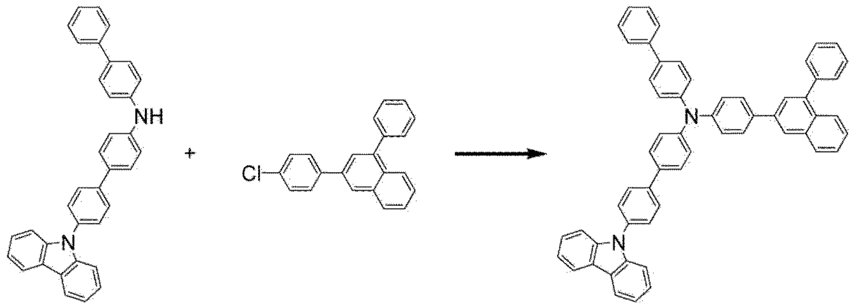

(60)

The structure of the obtained white powder was confirmed using NMR analysis.

The following signals corresponding to 40 protons were detected in the $^1H$-NMR($CDCl_3$) spectrum:

δ(ppm)=8.16(2H), 8.07(1H), 7.96(1H), 7.91(1H), 7.82(2H), 7.73(2H), 7.70(1H), 7.62(6H), 7.56(4H), 7.54-7.39(11H), 7.31(9H).

[EXAMPLE 7]

<Synthesis of Compound (30)>

In a nitrogen-purged reaction vessel, a mixture of N-phenyl-4-(3-phenylnaphthalen-1-yl)aniline (6.7g), 9-(4'-chloro-[1,1'-biphenyl]-3-yl)-carbazole (7.0g), t-butoxide sodium: 2.6g, bis[tri(t-butylphosphine)]palladium(0) (0.2g), and toluene (70ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was separated via column chromatography using silica gel as the stationary phase and a dichloromethane/n-heptane mixture as the eluent. This process afforded Compound (30) as a

white powder (10.1g, yield: 81.3%).

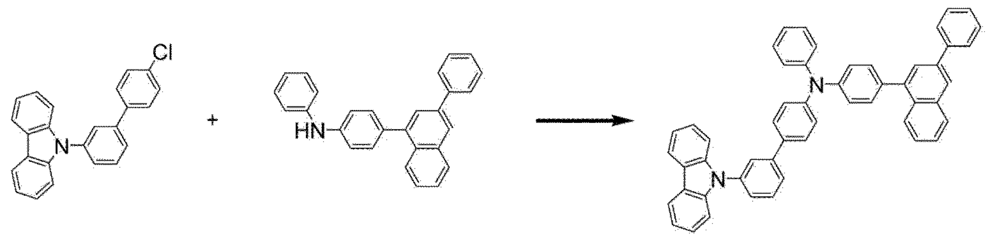

(30)

The structure of the obtained white powder was confirmed using NMR analysis.

The following signals corresponding to 36 protons were detected in the $^1$H-NMR($CDCl_3$) spectrum:

δ(ppm)=8.19(2H), 8.08-8.04(2H), 7.99(1H), 7.83-7.67(6H), 7.61(2H), 7.57-7.27(22H), 7.13(1H).

[EXAMPLE 8]

<Synthesis of Compound (31)>

In a nitrogen-purged reaction vessel, a mixture of N-{4-(3-phenylnaphthalen-1-yl)phenyl}-[1,1'-biphenyl]-4-amine (8.0g), 9-(4'-chloro-[1,1'-biphenyl]-3-yl)-carbazole (7.0g), t-butoxide sodium (2.6g), bis[tri(t-butylphosphine)]palladium(0) (0.2g), and toluene (80ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was separated via column chromatography using silica gel as the stationary phase and a dichloromethane/n-heptane mixture as the eluent. This process afforded Compound (31) as a white powder (2.7g, yield: 19.7%).

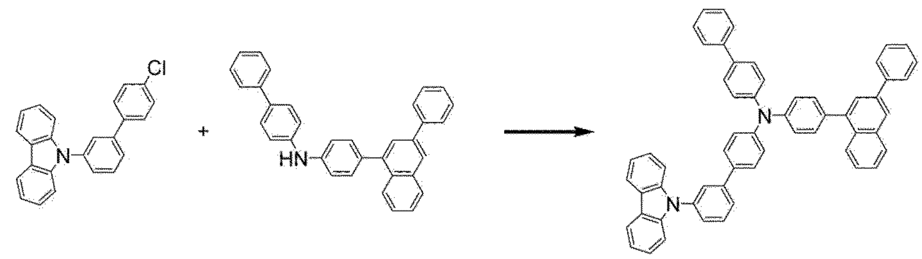

(31)

The structure of the obtained white powder was confirmed using NMR analysis.

The following signals corresponding to 40 protons were detected in the $^1$H-NMR($CDCl_3$) spectrum:

δ(ppm)=8.20(2H), 8.08-8.06(2H), 8.00(1H), 7.85-7.40(26H), 7.37-7.32(9H).

[EXAMPLE 9]

<Synthesis of Compound (46)>

In a nitrogen-purged reaction vessel, a mixture of N-{4-(4-phenylnaphthalen-2-yl)phenyl}-[1,1'-biphenyl]-2-amine (8.0g), 9-(4'-chloro-[1,1'-biphenyl]-3-yl)-carbazole (7.6g), t-butoxide sodium (3.4g), bis[tri(t-butylphosphine)]palladium(0) (0.2g), and toluene (160ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was separated via column chromatography using silica gel as the stationary phase and a dichloromethane/n-heptane mixture as the eluent. This process afforded Compound (46) as a white powder (9.9g, yield: 72.4%).

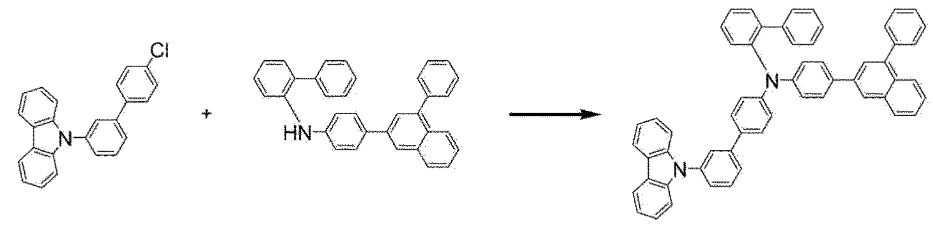

(46)

The structure of the obtained white powder was confirmed using NMR analysis.

The following signals corresponding to 40 protons were detected in the $^1$H-NMR($CDCl_3$) spectrum:

δ(ppm)=8.19(2H), 8.00(1H), 7.95(1H), 7.90(1H), 7.74(1H), 7.67-7.64(3H), 7.58-7.31(22H), 7.22(2H), 7.17-7.11(3H), 7.01(4H).

[EXAMPLE 10]

<Synthesis of Compound (85)>

In a nitrogen-purged reaction vessel, a mixture of 3'-(carbazole-9-yl)-N-phenyl-[1,1'biphenyl]-4-amine (10.0g), 2-(4-chlorophenyl)-6-phenyl-naphthalene (8.1g), t-butoxide sodium (3.5g), t-butylphosphine (50wt% toluene solvent) (0.4g), tris(dibenzylidene)acetone dipalladium(0) (0.5g), and xylene (100ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was subjected to purification by precipitation using toluene/acetone mixture solvent to afford Compound (85) as a pale yellow powder (9.8g, yield: 58.4%).

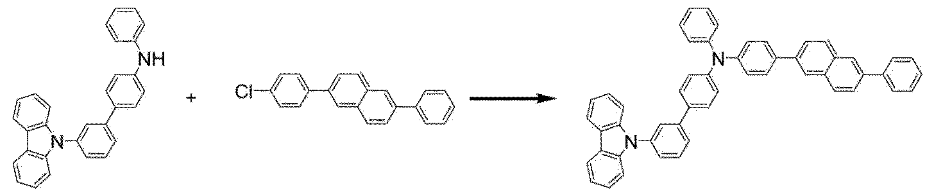

(85)

The structure of the obtained pale yellow powder was confirmed using NMR analysis.

The following signals corresponding to 36 protons were detected in the $^1$H-NMR($CDCl_3$) spectrum:

δ(ppm)=8.20(2H), 8.08(2H), 7.98(2H), 7.82(2H), 7.79(1H), 7.77(2H), 7.74-7.66(4H), 7.59(2H), 7.57-7.50(5H), 7.46 (2H), 7.42(1H), 7.34(4H), 7.28-7.23(6H), 7.12(1H).

[EXAMPLE 11]

<Synthesis of Compound (96)>

In a nitrogen-purged reaction vessel, a mixture of N-{4-(2-phenylnaphthalen-7-yl)phenyl}-[1,1'-biphenyl]-4-amine (10.0g), 9-(4'-chloro-[1,1'-biphenyl]-2-yl)-carbazole (8.7g), t-butoxide sodium (3.2g), t-butylphosphine(50wt% toluene solution) (0.4g), tris(dibenzylidene)acetone dipalladium(0) (0.4g), and xylene (100ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was separated via column chromatography using silica gel as the stationary phase and a dichloromethane/n-heptane mixture as the eluent. This process afforded Compound (96) as a pale yellow powder (96) (7.9g, yield: 46.2%).

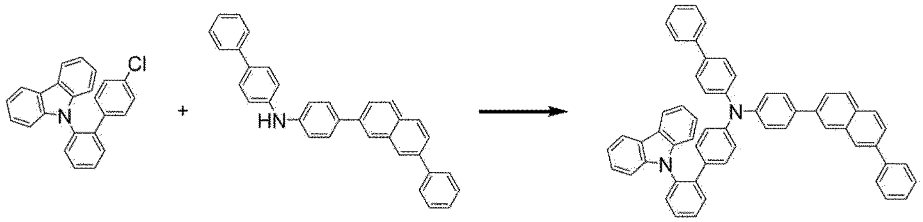

(96)

The structure of the obtained pale yellow powder was confirmed using NMR analysis.

The following signals corresponding to 40 protons were detected in the $^1$H-NMR($CDCl_3$) spectrum:

δ(ppm)=8.12(2H), 8.10(2H), 7.95(2H), 7.76(5H), 7.67-7.50(9H), 7.50-7.39(5H), 7.34(3H), 7.28(2H), 7.13(2H), 6.95 (1H), 6.92(2H), 6.89(2H), 6.86(1H), 6.77(2H).

[EXAMPLE 12]

<Synthesis of Compound (245)>

In a nitrogen-purged reaction vessel, a mixture of N-phenyl-4-(4-phenylnaphthalen-1-yl)-aniline (10.0g), 9-(4'-chloro-[1,1'-biphenyl]-3-yl)-carbazole (9.5g), t-butoxide sodium (5.2g), t-butylphosphine (50wt% toluene solution) (0.4g), tris(dibenzylidene)acetone dipalladium(0) (0.5g), and xylene (100ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was separated via column chromatography using silica gel as the stationary phase and a dichloromethane/n-heptane mixture as the eluent. This process afforded Compound (245) as a pale yellow powder (8.5g, yield: 45.8%).

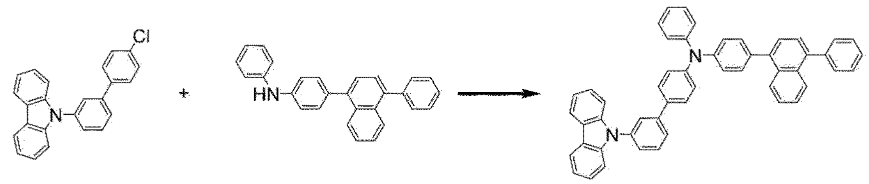

(245)

The structure of the obtained pale yellow powder was confirmed using NMR analysis.

The following signals corresponding to 36 protons were detected in the $^1$H-NMR((DMSO) spectrum:

δ(ppm)=8.27(2H), 8.01(1H), 7.89-7.84(3H), 7.80-7.74(3H), 7.60-7.45(16H), 7.41(2H), 7.32(2H), 7.23-7.14(7H).

[EXAMPLE 13]

<Synthesis of Compound (247)>

In a nitrogen-purged reaction vessel, a mixture of N-{4-(4-phenylnaphthalen-1-yl)phenyl}-dibenzofuran-3-amine (14.0g), 9-(4'-chloro-[1,1'-biphenyl]-3-yl)-carbazole (11.8g), t-butoxide sodium (3.5g), t-butylphosphine (50wt% toluene solution) (0.5g), tris(dibenzylidene)acetone dipalladium(0) (0.6g), and xylene (140ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. Purification by precipitation in toluene/acetone mixture solvent afforded Compound (247) as a pale yellow powder (13.4g, yield: 56.7%).

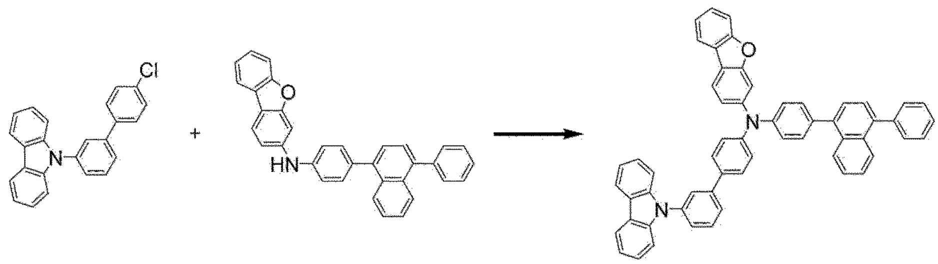

(247)

The structure of the obtained pale yellow powder was confirmed using NMR analysis.

The following signals corresponding to 38 protons were detected in the $^1$H-NMR($CDCl_3$) spectrum:

δ(ppm)=8.27(2H), 8.13-8.08(2H), 8.03(1H), 7.91-7.76(6H), 7.66(1H), 7.61-7.38(19H), 7.33-7.23(7H).

[EXAMPLE 14]

<Synthesis of Compound (249)>

In a nitrogen-purged reaction vessel, a mixture of N-phenyl-4-{4-(naphthalen-2-yl)naphthalen-1-yl}-aniline (10.0g), 9-(4'-chloro-[1,1'-biphenyl]-3-yl)-carbazole (9.2g), t-butoxide sodium: 3.4g, t-butylphosphine (50wt% toluene solution) (0.4g), tris(dibenzylidene)acetone dipalladium(0) (0.4g), and xylene (100ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was separated via column chromatography using silica gel as the stationary phase and a dichloromethane/n-heptane mixture as the eluent. This process afforded Compound (249) as a white powder (7.3g, yield: 41.6%).

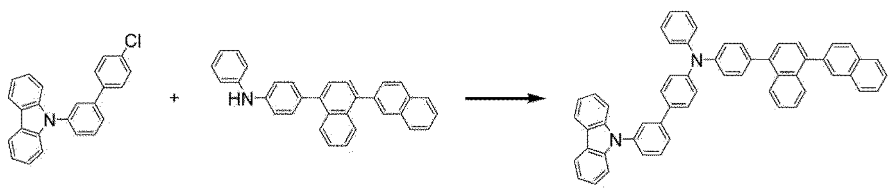

(249)

The structure of the obtained white powder was confirmed using NMR analysis.

The following signals corresponding to 38 protons were detected in the $^1$H-NMR($CDCl_3$) spectrum:

δ(ppm)=8.19(2H), 8.14(1H), 8.05-7.92(5H), 7.83(1H), 7.71(3H), 7.64-7.43(15H), 7.40-7.29(10H), 7.13(1H).

[EXAMPLE 15]

<Synthesis of Compound (252)>

In a nitrogen-purged reaction vessel, a mixture of N-phenyl-4-(1-phenylnaphthalen-5-yl)-aniline (9.0g), 9-(4'-chloro-[1,1'-biphenyl]-3-yl)-carbazole (9.0g), t-butoxide sodium (2.8g), t-butylphosphine (50wt% toluene solution) (0.4g), tris(dibenzylidene)acetone dipalladium(0) (0.4g), and xylene (90ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. Purification by recrystallization in toluene solvent afforded Compound (252) as a white powder (7.9g, yield: 47.3 %).

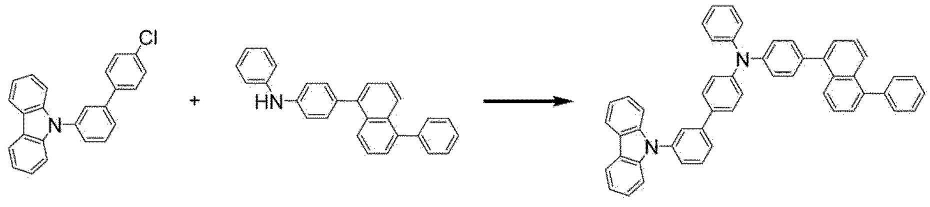

(252)

The structure of the obtained white powder was confirmed using NMR analysis.

The following signals corresponding to 36 protons were detected in the $^1$H-NMR($CDCl_3$) spectrum:

δ(ppm)=8.20(2H), 8.06(1H), 7.92(1H), 7.84(1H), 7.73(1H), 7.69(1H), 7.62(2H), 7.58-7.43(16H), 7.40-7.28(10H), 7.13 (1H).

[EXAMPLE 16]

<Synthesis of Compound (256)>

In a nitrogen-purged reaction vessel, a mixture of 3'-(carbazole-9-yl)-N-phenyl-[1,1'biphenyl]-4-amine (7.5g), 7-(4-chlorophenyl)-1-phenyl-naphthalene (5.9g), t-butoxide sodium (3.5g), t-butylphosphine (50wt% toluene solution) (0.3g), tris(dibenzylidene)acetone dipalladium(0) (0.3g), and xylene (75ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. Purification by recrystallization in toluene solvent afforded Compound (256) as a white powder (6.9g, yield: 58.5%).

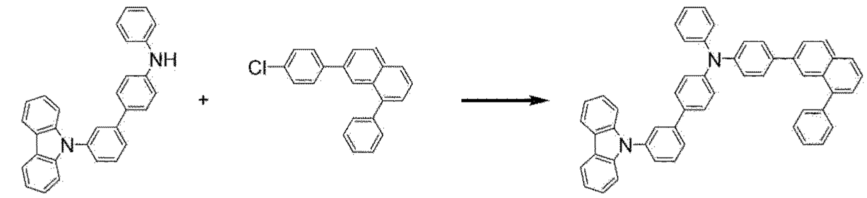

(256)

The structure of the obtained white powder was confirmed using NMR analysis.

The following signals corresponding to 36 protons were detected in the $^1$H-NMR($CDCl_3$) spectrum:

δ(ppm)=8.19(2H), 8.11(1H), 7.99(1H), 7.89(1H), 7.80-7.76(2H), 7.72-7.65(2H), 7.58-7.43(16H), 7.35-7.29(4H), 7.22-7.18(6H), 7.09(1H).

[EXAMPLE 17]

<Synthesis of Compound (258)>

In a nitrogen-purged reaction vessel, a mixture of N-phenyl-4-(1-phenylnaphthalen-6-yl)-aniline (7.8g), 9-(4'-chloro-[1,1'-biphenyl]-3-yl)-carbazole (8.2g), t-butoxide sodium (3.0g), t-butylphosphine (50wt% toluene solution) (0.4g), tris(dibenzylidene)acetone dipalladium(0) (0.4g), and xylene (80ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. The resulting crude mixture was separated via column chromatography using silica gel as the stationary phase and a dichloromethane/n-heptane mixture as the eluent. This process afforded Compound (258) as a white powder (11.6g, yield: 80.2%).

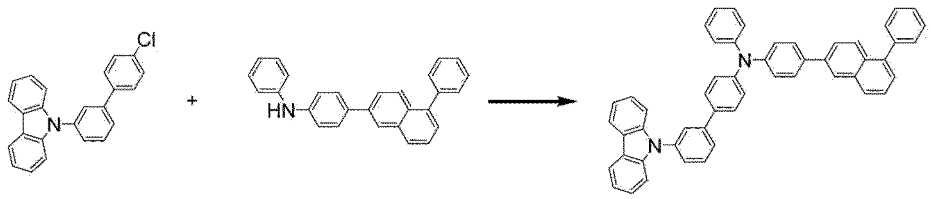

(258)

The structure of the obtained white powder was confirmed using NMR analysis.
The following signals corresponding to 36 protons were detected in the $^1$H-NMR(DMSO) spectrum:

δ(ppm)=8.27(3H), 8.02(1H), 7.88-7.74(9H), 7.63-7.37(14H), 7.33-7.29(2H), 7.12- 7.19(7H).

[EXAMPLE 18]

<Synthesis of Compound (261)>

In a nitrogen-purged reaction vessel, a mixture of N-([1,1'biphenyl]-4-yl)-2'-(carbazole-9-yl)-[1,1'biphenyl]-4-amine (7.5g), 1-(4-chlorophenyl)-7-phenyl-naphthalene (5.0g), t-butoxide sodium (3.0g), t-butylphosphine(50wt% toluene solution) (0.2g), tris(dibenzylidene)acetone dipalladium(0) (0.3g), and xylene (170ml) was stirred overnight under reflux while heating. Then, silica gel was added to the reaction mixture, followed by stirring for 30 minutes. Afterward, the mixture was filtered through Celite at 90°C, and the solvent was removed by vacuum distillation. Purification by precipitation in dichloromethane/acetone mixture solvent afforded Compound (261) as a white powder (5.5g, yield: 46.8%).

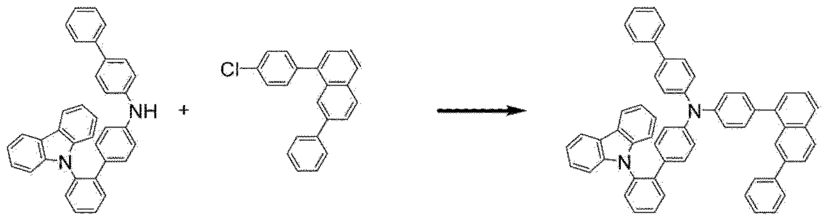

(261)

The structure of the obtained white powder was confirmed using NMR analysis.
The following signals corresponding to 40 protons were detected in the $^1$H-NMR($CDCl_3$) spectrum:

δ(ppm)=8.21(1H), 8.08(2H), 8.01(1H), 7.89(1H), 7.80-7.75(2H), 7.67-7.31(21H), 7.24(2H), 7.12(2H), 6.95-6.92(4H), 6.88(2H), 6.81(2H).

[EXAMPLE 19]

For the arylamine compounds represented by General Formula (I), the melting point and glass transition temperature were measured using a high-sensitivity differential scanning calorimeter (Bruker AXS, DSC3100SA). The results are presented in Table 1.

TABLE 1

| | Melting Point | Glass transition temperature |
|---|---|---|
| Compound 2 | - | 125°C |
| Compound 21 | - | 113°C |
| Compound 22 | - | 124°C |
| Compound 42 | 253°C | 125°C |
| Compound 43 | - | 129°C |

(continued)

| | Melting Point | Glass transition temperature |
|---|---|---|
| Compound 60 | - | 138°C |
| Compound 30 | - | 120°C |
| Compound 31 | - | 131°C |
| Compound 46 | - | 125°C |
| Compound 85 | - | 117°C |
| Compound 96 | - | 121°C |
| Compound 245 | - | 121°C |
| Compound 247 | - | 134°C |
| Compound 249 | - | 128°C |
| Compound 252 | - | 121°C |
| Compound 256 | - | 120°C |
| Compound 258 | - | 117°C |
| Compound 261 | - | 121°C |

The arylamine compounds represented by General Formula (I) from Examples 1 to 18 exhibited glass transition temperatures above 100°C, indicating their stability in thin-film states.

[EXAMPLE 20]

Using the arylamine compounds represented by General Formula (I) from Examples 1 to 18, films with a thickness of 100 nm were deposited on an ITO substrate. The work function was measured using an ionization potential measurement device (Sumitomo Chemical Co., Ltd., PYS-202). The results are shown in Table 2.

TABLE 2

| | Work function |
|---|---|
| Compound 2 | 5.67eV |
| Compound 21 | 5.64eV |
| Compound 22 | 5.62eV |
| Compound 42 | 5.67eV |
| Compound 43 | 5.68eV |
| Compound 60 | 5.68eV |
| Compound 30 | 5.70eV |
| Compound 31 | 5.66eV |
| Compound 46 | 5.68eV |
| Compound 85 | 5.73eV |
| Compound 96 | 5.64eV |
| Compound 245 | 5.68eV |
| Compound 247 | 5.68eV |
| Compound 249 | 5.69eV |
| Compound 252 | 5.69eV |
| Compound 256 | 5.69eV |
| Compound 258 | 5.66eV |

(continued)

| | Work function |
|---|---|
| Compound 261 | 5.65eV |

The arylamine compounds represented by General Formula (I) from Examples 1 to 18 exhibit suitable energy levels when compared to the work function of 5.4eV, which is typical for common hole-transport materials such as NPD and TPD. These compounds demonstrate excellent hole-transport capabilities.

The arylamine compounds of the present disclosure are effective as materials for the hole-injection layer, hole-transport layer, electron-blocking layer, or emission layer in organic EL devices. They provide improvements in the light-emission efficiency, driving voltage, and durability of conventional organic EL devices.

[EXAMPLE 21]

An organic EL device was fabricated, as shown in FIG. 22, by sequentially depositing a hole injection layer 3, a hole transport layer 4, an electron blocking layer 5, an emission layer 6, an electron transport layer 7, an electron injection layer 8, a cathode 9, and a capping layer 10 on a glass substrate 1 with a pre-formed transparent anode 2 consisting of a reflective ITO electrode.

Specifically, a glass substrate 1 on which a 50 nm thick ITO layer, a 100 nm thick reflective layer of silver alloy, and a 5 nm thick ITO layer were sequentially formed was ultrasonically cleaned in isopropyl alcohol for 20 minutes, then dried on a hot plate at 250°C for 10 minutes. Subsequently, the glass substrate with the ITO layer underwent UV ozone treatment for 2 minutes. The substrate was then placed in a vacuum deposition chamber which was then evacuated to a pressure below 0.001 Pa. Next, the electron acceptor (Acceptor-1) and compound (HTM-1) of the following structural formulas were co-deposited at a deposition ratio of Acceptor-1:Compound (HTM-1) = 3:97 to form a hole injection layer 3 10 nm in thickness, covering the transparent anode 2. On top of the hole injection layer 3, a hole transport layer 4 with a thickness of 140 nm was formed using Compound (HTM-1). On the hole transport layer 4, an electron blocking layer 5 with a thickness of 5 nm was formed of Compound 2 from Example 1. On the electron blocking layer 5, Compound (EMD-1) and Compound (EMH-1) of the following structural formulas were co-deposited at a deposition rate ratio of EMD-1:EMH-1 = 5:95 to form an emission layer 6 with a thickness of 20 nm. An electron transport layer 7 was subsequently formed on top of the emission layer 6 by co-depositing Compound (ETM-1) and Compound (ETM-2) at a deposition rate ratio of ETM-1:ETM-2 = 50:50, with a thickness of 30 nm. On the electron transport layer 7, an electron injection layer 8 was formed using lithium fluoride to a thickness of 1 nm. Next, a cathode 9 was formed using a magnesium-silver alloy to a thickness of 12 nm on the electron injection layer 8. Finally, a capping layer 10 was formed using Compound (CPL-1), with a thickness of 60 nm. The fabricated organic EL device was evaluated for its characteristics in ambient conditions at room temperature. The emission characteristics under DC voltage application were summarized and presented in Table 3.

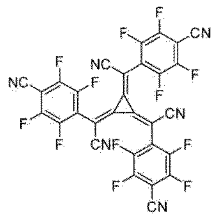
(Acceptor-1)

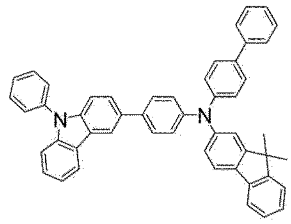
(HTM-1)

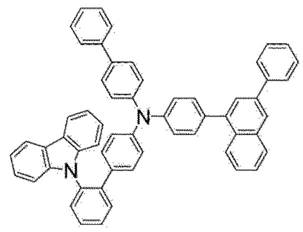
(2)

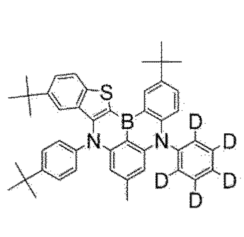
(EMD-1)

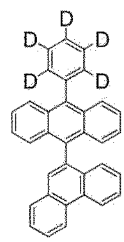
(EMH-1)

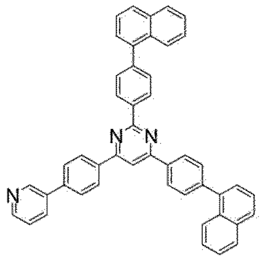

(ETM-1)

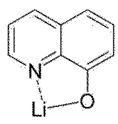

(ETM-2)

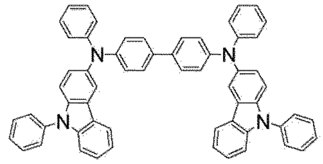

(CPL-1)

[EXAMPLE 22]

An organic EL device was fabricated in the same manner as in Example 21, with the exception of using the compound (21) of Example 2, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 23]

An organic EL device was fabricated in the same manner as in Example 21, with the exception of using the compound (22) of Example 3, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 24]

An organic EL device was fabricated in the same manner as in Example 21, with the exception of using the compound (42) of Example 4, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 25]

An organic EL device was fabricated in the same manner as in Example 21, with the exception of using the compound (43) of Example 5, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 26]

An organic EL device was fabricated in the same manner as in Example 21, with the exception of using the compound (60) of Example 6, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 27]

An organic EL device was fabricated in the same manner as in Example 21, with the exception of using the compound (30) of Example 7, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 28]

An organic EL device was fabricated in the same manner as in Example 21, with the exception of using the compound (31) of Example 8, instead of the compound (2) of Example 1, as a material for electron blocking layer (5). The fabricated organic EL device was subjected to performance testing at room temperature in ambient air. The luminous

properties were measured by applying a DC voltage to the device. The measurement results are summarized in Table 3.

[EXAMPLE 29]

An organic EL device was fabricated in the same manner as in Example 21, with the exception of using the compound (46) of Example 9, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 30]

An organic EL device was fabricated in the same manner as in Example 21, with the exception of using the compound (85) of Example 10, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 31]

An organic EL device was fabricated in the same manner as in Example 21, with the exception of using the compound (96) of Example 11, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 32]

An organic EL device was fabricated in the same manner as in Example 21, with the exception of using the compound (245) of Example 12, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 33]

An organic EL device was fabricated in the same manner as in Example 21, with the exception of using the compound (247) of Example 13, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 34]

An organic EL device was fabricated in the same manner as in Example 21, with the exception of using the compound (249) of Example 14, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 3 5]

An organic EL device was fabricated in the same manner as in Example 21, with the exception of using the compound (252) of Example 15, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 36]

An organic EL device was fabricated in the same manner as in Example 21, with the exception of using the compound (256) of Example 16, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 37]

An organic EL device was fabricated in the same manner as in Example 21, with the exception of using the compound (258) of Example 17, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[EXAMPLE 38]

An organic EL device was fabricated in the same manner as in Example 21, with the exception of using the compound (261) of Example 18, instead of the compound (2) of Example 1, as a material for electron blocking layer (5). The fabricated organic EL device was subjected to performance testing at room temperature in ambient air. The luminous properties were measured by applying a DC voltage to the device. The measurement results are summarized in Table 3.

[COMPARATIVE EXAMPLE 1]

For comparison, an organic EL device was fabricated in the same manner as in Example 21, with the exception of using the compound (HTM-2) of the following structural formula, instead of the compound (2) of Example 1, as a material for the electron blocking layer (5) (see Patent literature 7). The fabricated organic EL device was tested for its properties at room temperature in ambient air. The luminous properties were measured by applying a DC voltage to the device. The results of the measurements are summarized in Table 3.

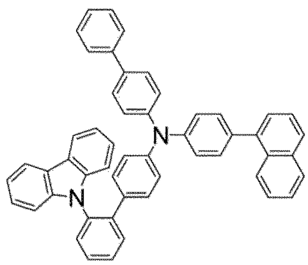

(HTM-2)

[COMPARATIVE EXAMPLE 2]

For comparison, an organic EL device was fabricated in the same manner as in Example 21, with the exception of using the compound (HTM-3) of the following structural formula, instead of the compound (2) of Example 1, as a material for the electron blocking layer (5) (see Patent literature 8). The fabricated organic EL device was tested for its properties at room temperature in ambient air. The luminous properties were measured by applying a DC voltage to the device. The results of the measurements are summarized in Table 3.

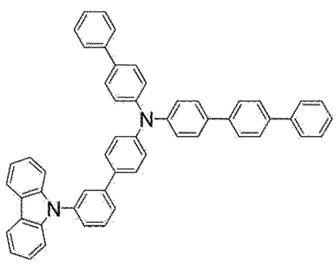

(HTM-3)

[COMPARATIVE EXAMPLE 3]

For comparison, an organic EL device was fabricated in the same manner as in Example 21, with the exception of using the compound (HTM-4) of the following structural formula, instead of the compound (2) of Example 1, as a material for the electron blocking layer (5) (see Patent literature 9). The fabricated organic EL device was tested for its properties at room temperature in ambient air. The luminous properties were measured by applying a DC voltage to the device. The

results of the measurements are summarized in Table 3.

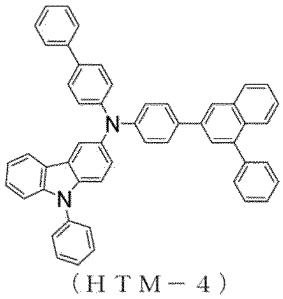

(HTM-4)

The results of measuring the lifetime of organic EL devices fabricated in Examples 21 to 38 and Comparative Examples 1 to 3 are summarized in Table 3. The device lifetime was determined as the time it took for the initial luminance of 2000 cd/$m^2$ to decrease to 1900 cd/$m^2$ (corresponding to 95% of the initial luminance, decay to 95%) under constant current operation.

TABLE 3

| | Electron blocking layer | Volt. [V] (@10mA/cm2) | Brightness [cd/m2] (@10mA/cm2) | Luminous efficiency (@10mA/cm2) | Power efficiency (@10mA/cm2) | Device Lifetime (decay to 95%) |
|---|---|---|---|---|---|---|
| Ex. 21 | Cpd. 2 | 3. | 1041 | 10.43 | 10.08 | 453 hours |
| Ex. 22 | Cpd. 2 | 3. | 1032 | 10.36 | 10.02 | 475 hours |
| Ex. 23 | Cpd. 2 | 3. | 1040 | 10.41 | 10.08 | 504 hours |
| Ex. 24 | Cpd. 2 | 3. | 989 | 9.89 | 9.62 | 433 hours |
| Ex. 25 | Cpd. 2 | 3. | 978 | 9.82 | 9.55 | 698 hours |
| Ex. 26 | Cpd. 2 | 3. | 962 | 9.84 | 9.41 | 423 hours |
| Ex. 27 | Cpd. 2 | 3. | 1025 | 10.26 | 9.73 | 557 hours |
| Ex. 28 | Cpd. 2 | 3. | 1009 | 10.11 | 9.79 | 707 hours |
| Ex. 29 | Cpd. 2 | 3. | 1001 | 10.02 | 9.58 | 510 hours |
| Ex. 30 | Cpd. 2 | 3. | 983 | 9.83 | 9.31 | 714 hours |
| Ex. 31 | Cpd. 2 | 3. | 989 | 9.90 | 9.54 | 456 hours |
| Ex. 32 | Cpd. 2 | 3. | 1013 | 10.13 | 9.71 | 609 hours |
| Ex. 33 | Cpd. 2 | 3. | 989 | 9.89 | 9.39 | 660 hours |
| Ex. 34 | Cpd. 2 | 3. | 1001 | 10.03 | 9.58 | 422 hours |
| Ex. 35 | Cpd. 2 | 3. | 1020 | 10.21 | 9.83 | 559 hours |
| Ex. 36 | Cpd. 2 | 3. | 10010 | 10.10 | 9.56 | 439 hours |
| Ex. 37 | Cpd. 2 | 3. | 1023 | 10.24 | 9.72 | 458 hours |
| Ex. 38 | Cpd. 2 | 3. | 1007 | 10.09 | 9.58 | 662 hours |
| C. Ex. 1 | HTM-2 | 3. | 909 | 9.11 | 8.53 | 328 hours |
| C. Ex. 2 | HTM-3 | 3. | 907 | 9.08 | 8.45 | 247 hours |
| C. Ex. 3 | HTM-4 | 3. | 883 | 8.84 | 8.68 | 253 hours |

As shown in Table 3, the luminous efficiency at a current density of 10 mA/$cm^2$ ranged from 9.82 to 10.42 cd/A for the organic EL devices in Examples 21 to 38, compared to 8.84 to 9.11 cd/A for the organic EL devices in Comparative Examples 1 to 3, indicating higher efficiency. Similarly, the power efficiency ranged from 9.39 to 10.08lm/W for the organic EL devices in Examples 21 to 38, compared to 8.45 to 8.68lm/W for those in Comparative Examples 1 to 3, again showing

superior performance. Furthermore, the device lifetime (defined as the time taken for luminance to decay to 95% of the initial luminance at 2000 cd/$m^2$) was extended significantly, from 247 to 328 hours for Comparative Examples 1 to 3, to 422 to 714 hours for Examples 21 to 38.

From these results, it is evident that the arylamine compounds with the specific structure represented by General Formula (I) in the present disclosure exhibit superior hole mobility and excellent electron-blocking capabilities compared to conventional arylamine compounds used as hole transport materials. Consequently, the organic EL devices utilizing the blue emission layer of the present disclosure achieve high luminous efficiency and extended lifetimes compared to conventional organic EL devices.

INDUSTRIAL APPLICABILITY

The organic EL devices employing the specific aryl amine compounds of the present disclosure not only enhance emission efficiency but also improve device durability, enabling their use in applications such as electric home appliances and lighting systems. Furthermore, the compounds described in this disclosure are also applicable in other electronic device fields, including electrophotographic photoreceptors, image sensors, photoelectric conversion devices, and solar cells.

[Description for Reference Numerals]

1: Glass substrate
2: Transparent anode
3: Hole injection layer
4: Hole transport layer
5: Electron blocking layer
6: Emission layer
7: Electron transport layer
8: Electron injection layer
9: Cathode
10: Capping layer

**Claims**

1. An arylamine compound represented by the following General Formula (I).

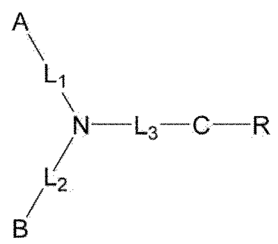

(I)

(wherein:

A represents a substituted or unsubstituted aromatic hydrocarbon group, a substituted or unsubstituted aromatic heterocyclic group, or a substituted or unsubstituted fused polycyclic aromatic group;
B represents a substituted or unsubstituted carbazolyl group;
C represents an unsubstituted naphthylene group;
R represents an unsubstituted aromatic hydrocarbon group, an unsubstituted aromatic heterocyclic group, or an unsubstituted fused polycyclic aromatic group; and
$L_1$ to $L_3$ represent a single bond, an unsubstituted divalent aromatic hydrocarbon group, an unsubstituted divalent aromatic heterocyclic group, or an unsubstituted divalent fused polycyclic aromatic group.)

2. The arylamine compound of claim 1, wherein C in the General Formula (I) is an unsubstituted 1,2-naphthylene group,

an unsubstituted 1,3-naphthylene group, an unsubstituted 2,4-naphthylene group, an unsubstituted 2,5-naphthylene group, an unsubstituted 2,6-naphthylene group, an unsubstituted 2,7-naphthylene group, or an unsubstituted 2,8-naphthylene group.

**3.** The arylamine compound of claim 2, wherein $L_2$ and $L_3$ in the General Formula (I) are an unsubstituted phenylene group or an unsubstituted biphenylene group.

**4.** The arylamine compound of claim 3, wherein R in the General Formula (I) is an unsubstituted phenyl group, an unsubstituted naphthyl group, an unsubstituted dibenzofuranyl group, an unsubstituted phenanthrenyl group, or an unsubstituted biphenyl group.

**5.** The arylamine compound of claim 4, wherein B in the General Formula (I) is a substituted or unsubstituted 9-carbazolyl group, a substituted or unsubstituted 2-carbazolyl group, or a substituted or unsubstituted 3-carbazolyl group.

**6.** The arylamine compound of claim 5, wherein B in the General Formula (I) is an unsubstituted 9-carbazolyl group.

**7.** An organic EL device, comprising a pair of electrodes and at least one organic layer interposed therebetween, wherein the organic layer comprises the arylamine compound as set forth in claim 1 or 2.

**8.** The organic EL device of claim 7, wherein the organic layer is a hole transport layer.

**9.** The organic EL device of claim 7, wherein the organic layer is an electron-blocking layer.

**10.** The organic EL device of claim 7, wherein the organic layer is a hole injection layer.

**11.** The organic EL device of claim 7, wherein the organic layer is an emission layer.

**12.** An electronic device comprising a pair of electrodes and at least one organic layer interposed therebetween, wherein the organic layer comprises the arylamine compound as set forth in claim 1 or 2.

# FIG. 1

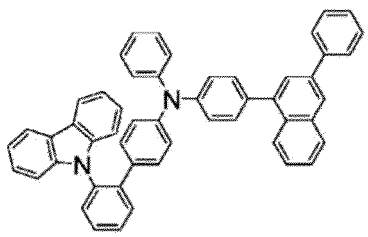

(1)

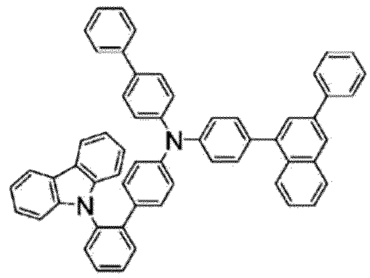

(2)

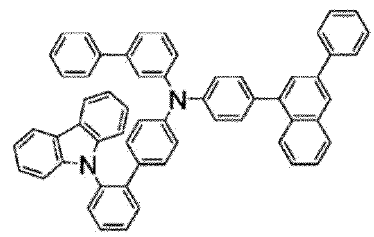

(3)

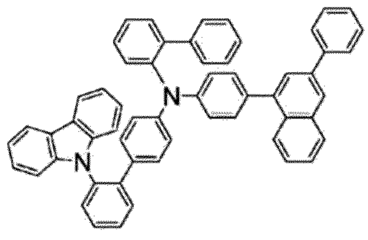

(4)

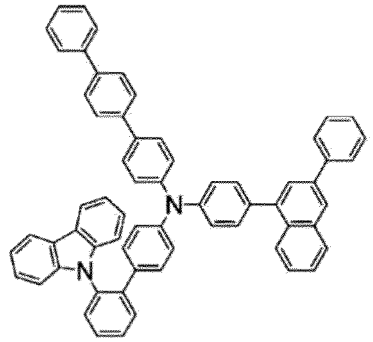

(5)

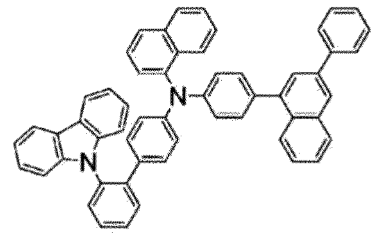

(6)

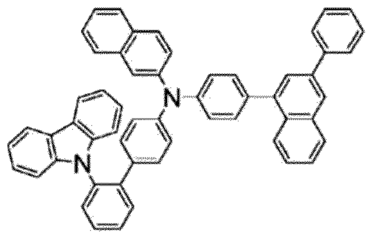

(7)

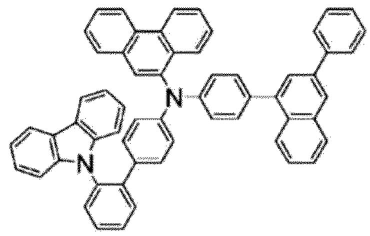

(8)

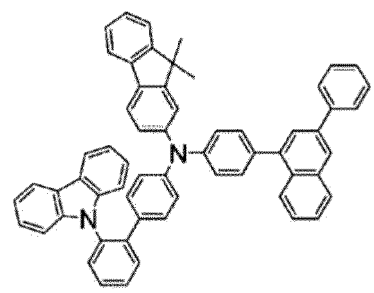

(9)

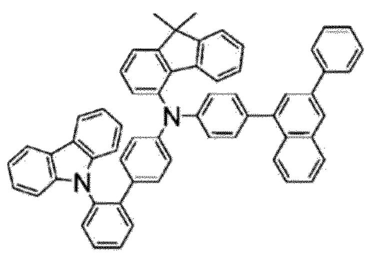

(10)

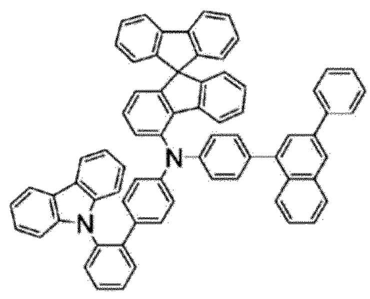

(11)

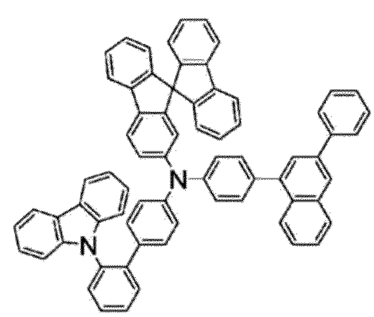

(12)

# FIG. 2

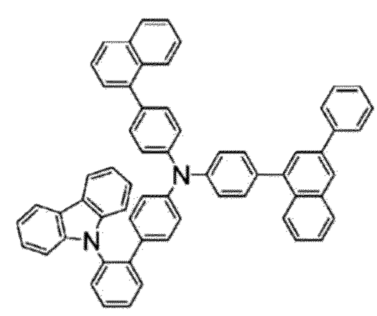

(13)

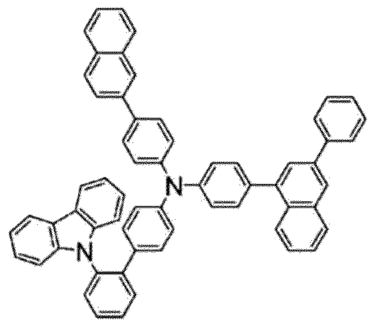

(14)

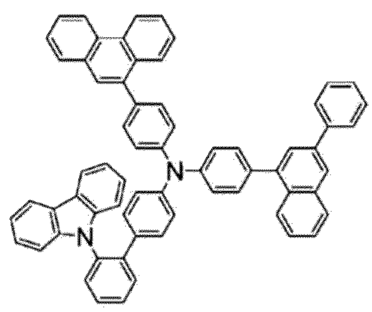

(15)

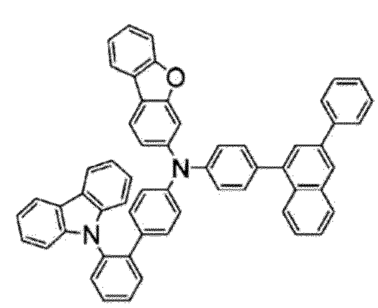

(16)

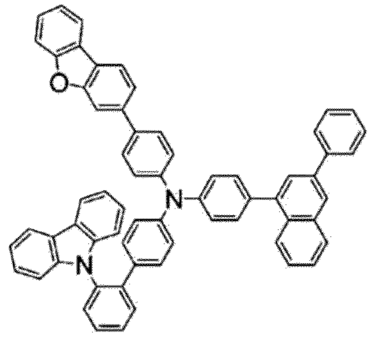

(17)

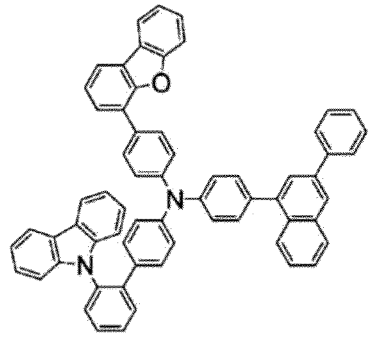

(18)

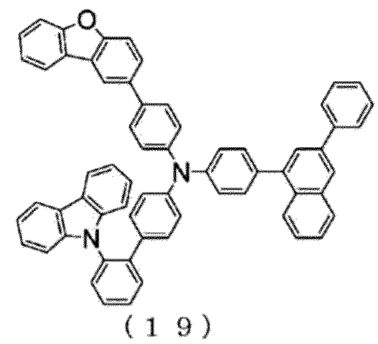

(19)

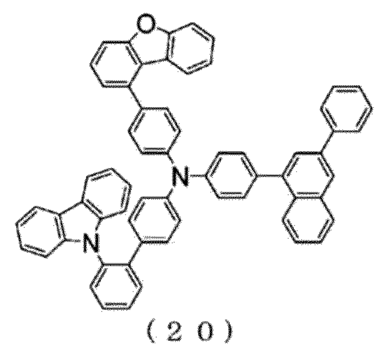

(20)

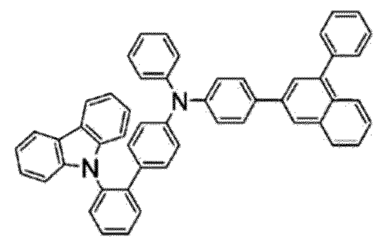

(21)

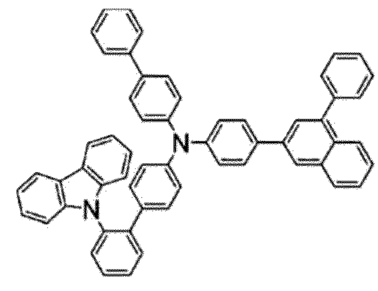

(22)

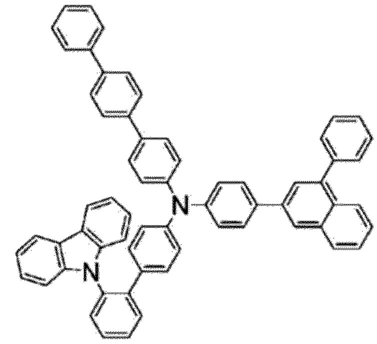

(23)

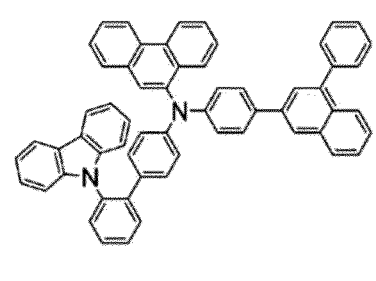

(24)

FIG. 3

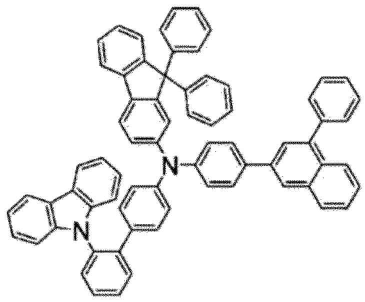

(25)

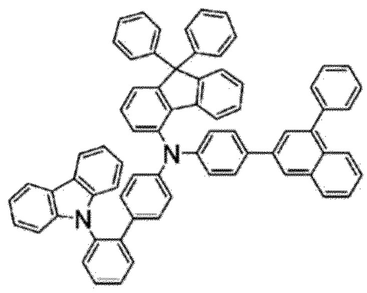

(26)

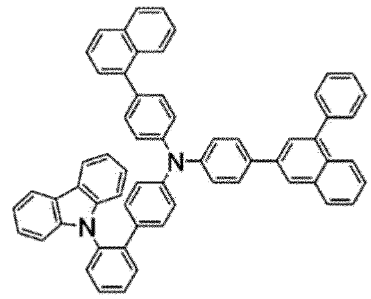

(27)

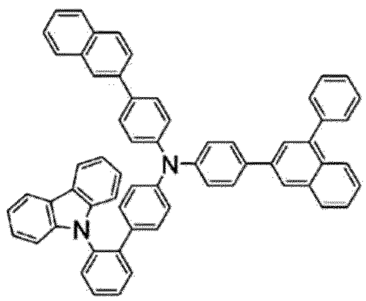

(28)

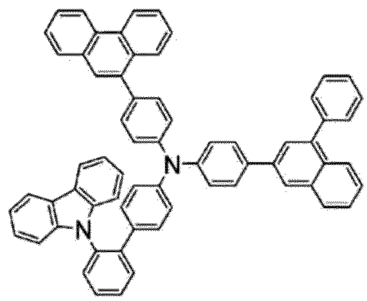

(29)

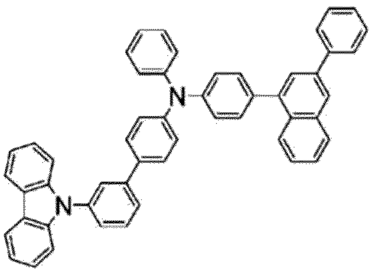

(30)

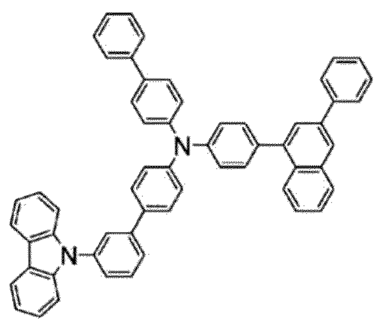

(31)

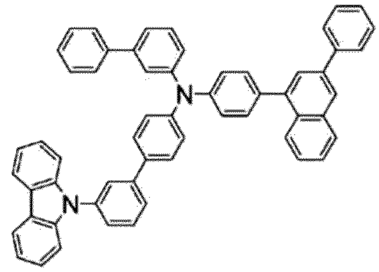

(32)

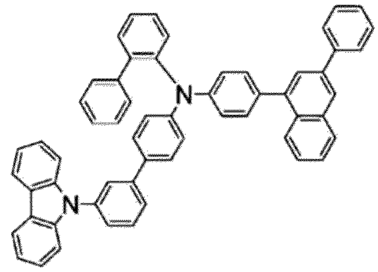

(33)

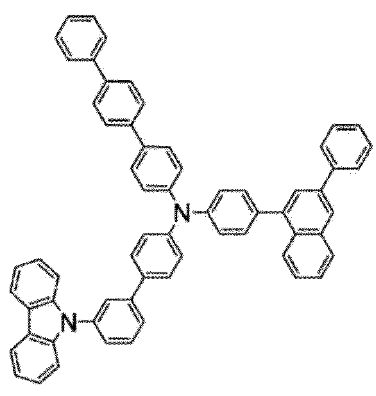

(34)

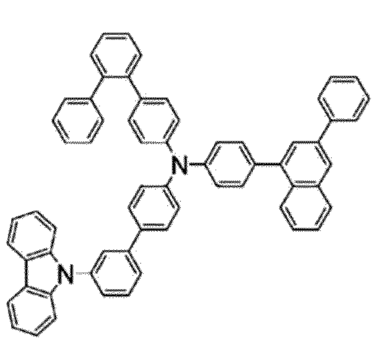

(35)

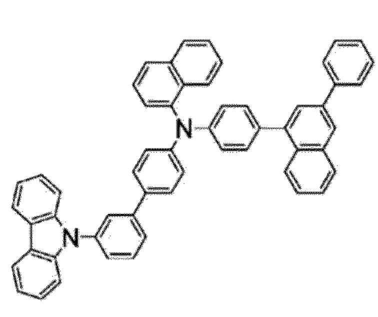

(36)

# FIG. 4

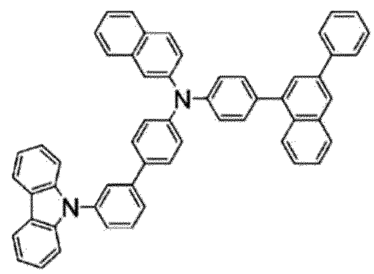

(37)

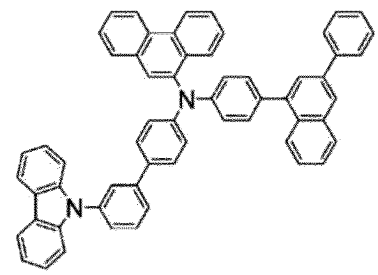

(38)

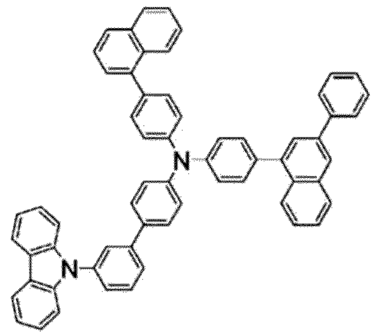

(39)

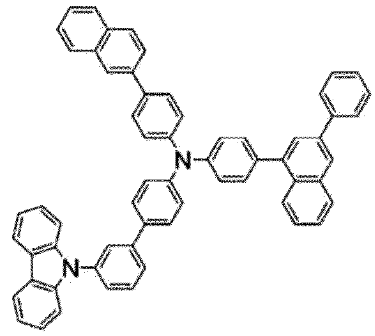

(40)

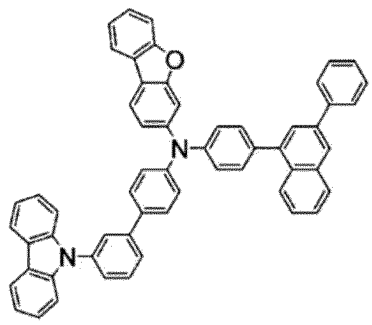

(41)

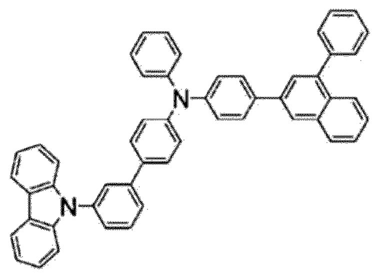

(42)

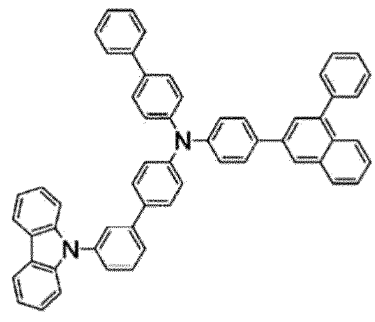

(43)

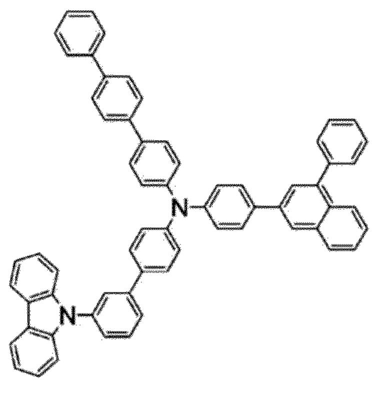

(44)

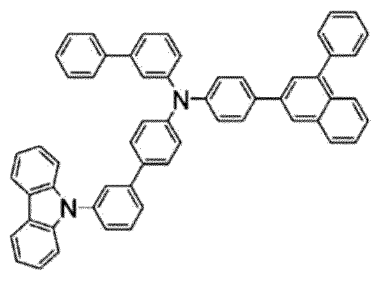

(45)

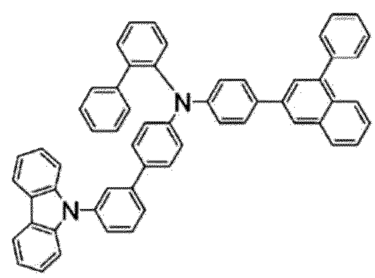

(46)

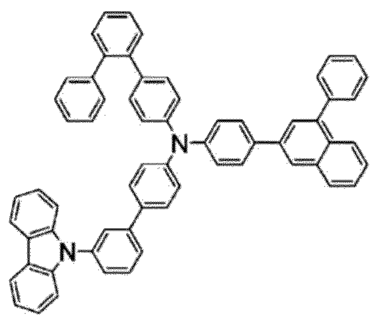

(47)

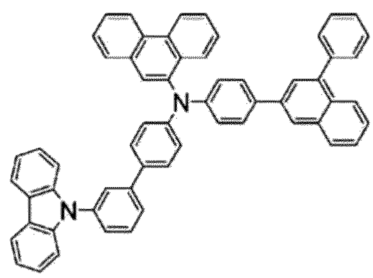

(48)

# FIG. 5

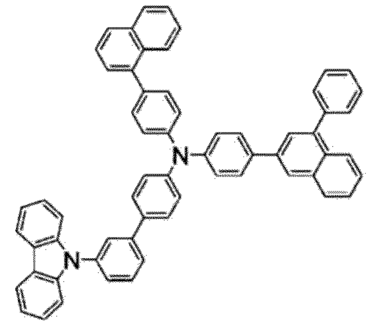

(49)

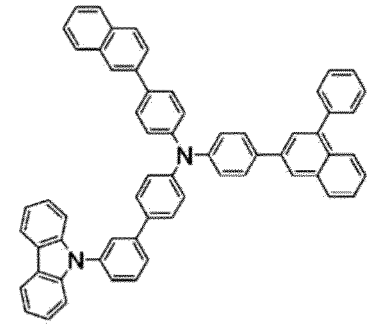

(50)

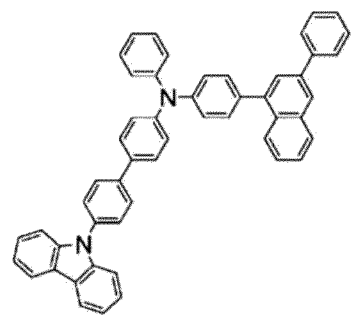

(51)

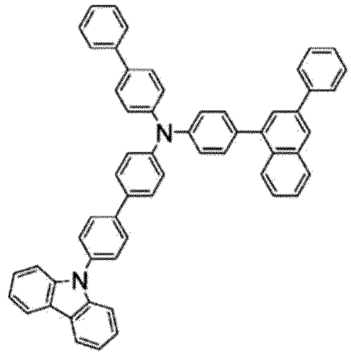

(52)

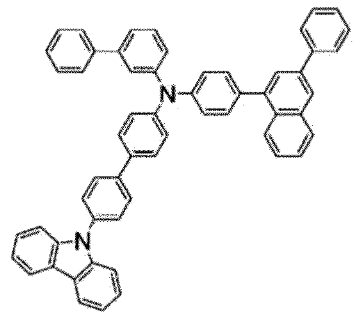

(53)

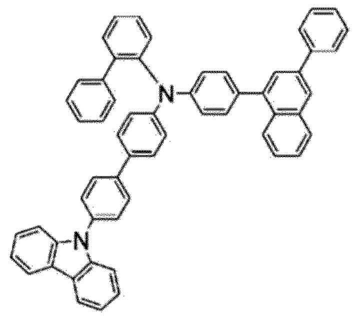

(54)

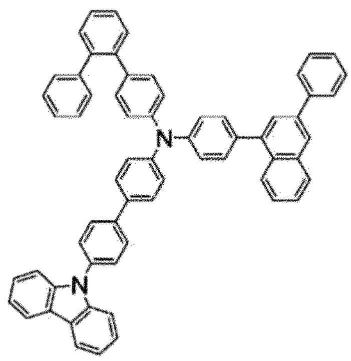

(55)

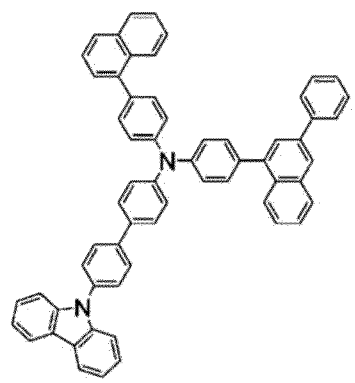

(56)

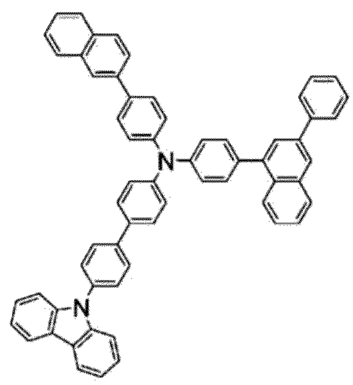

(57)

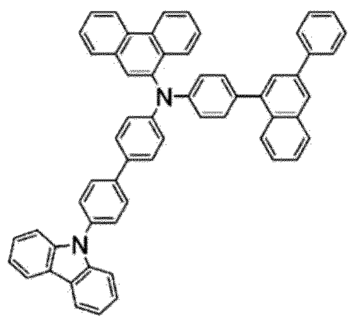

(58)

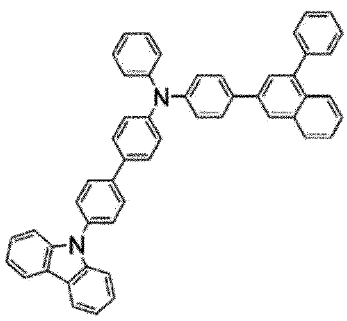

(59)

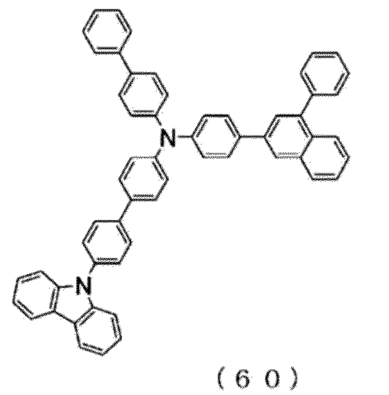

(60)

# FIG. 6

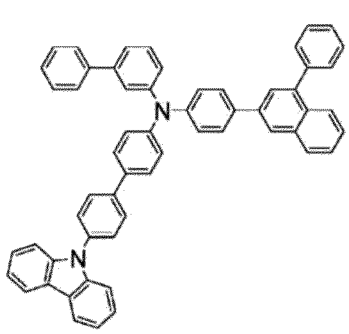

(61)

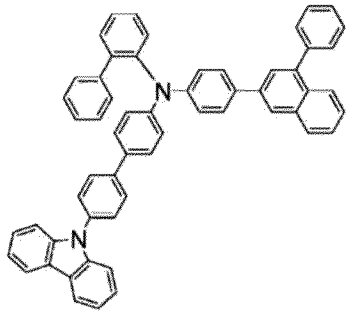

(62)

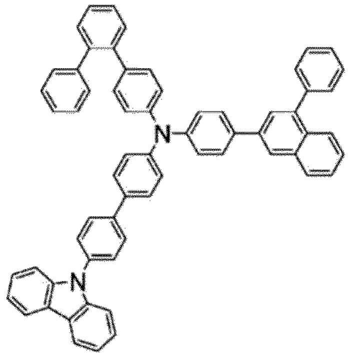

(63)

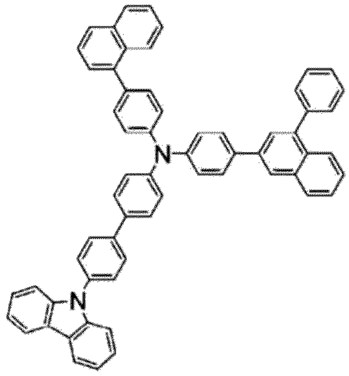

(64)

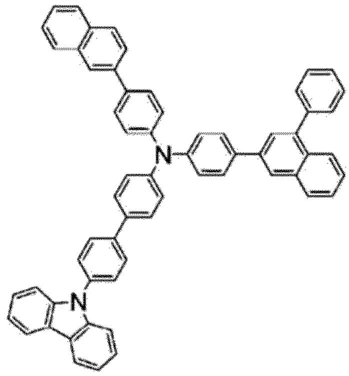

(65)

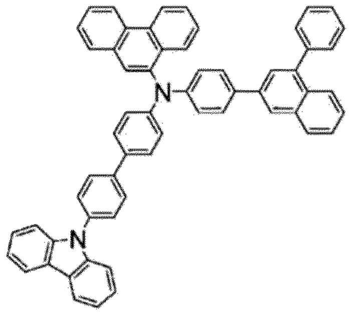

(66)

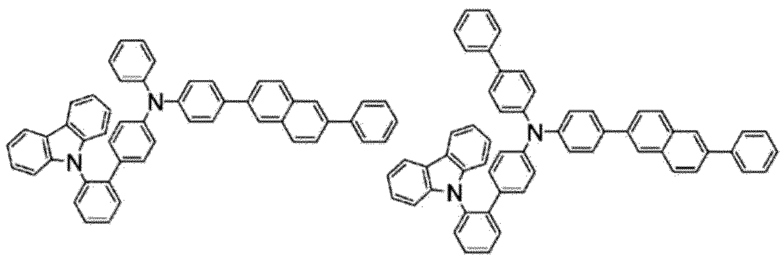

(67)

(68)

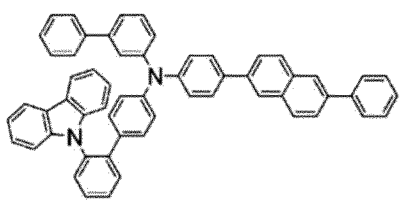

(69)

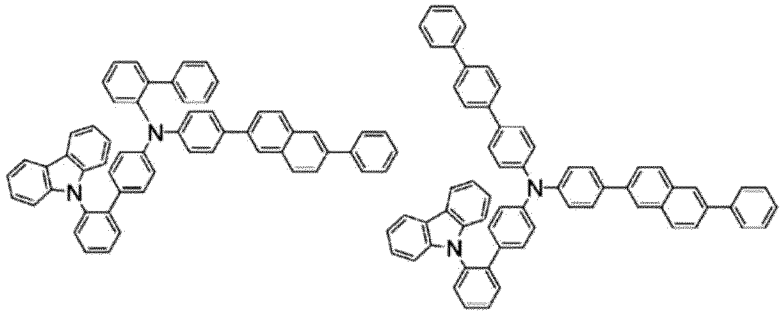

(70)

(71)

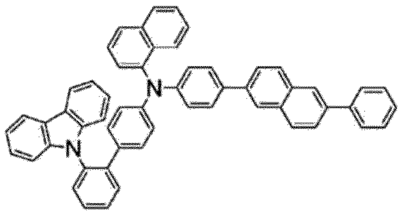

(72)

# FIG. 7

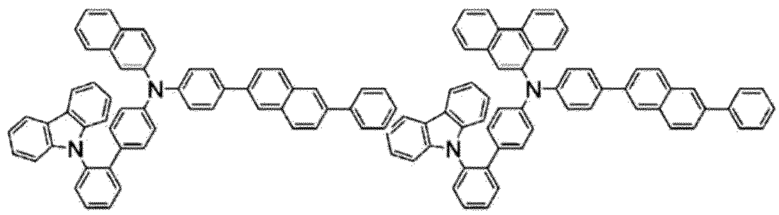

(7 3) (7 4)

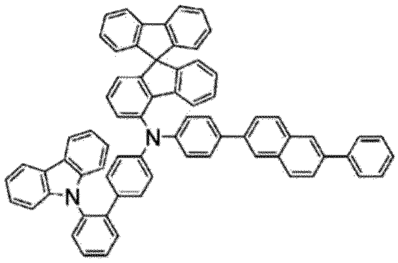

(7 5)

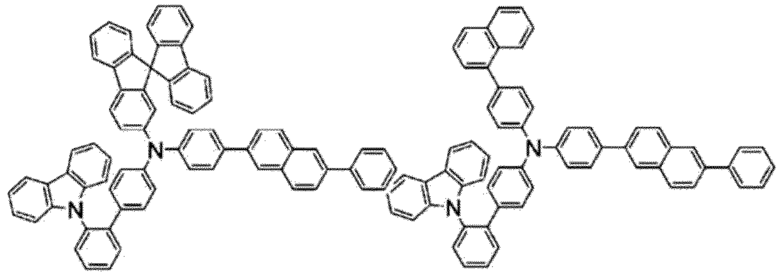

(7 6) (7 7)

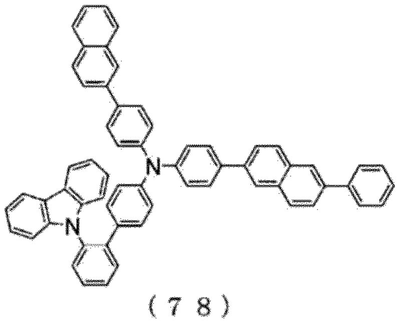

(7 8)

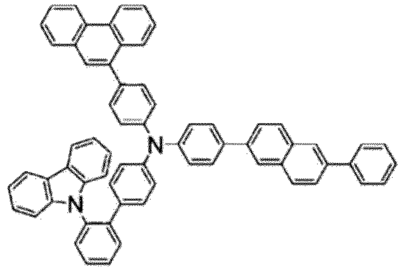

(7 9)

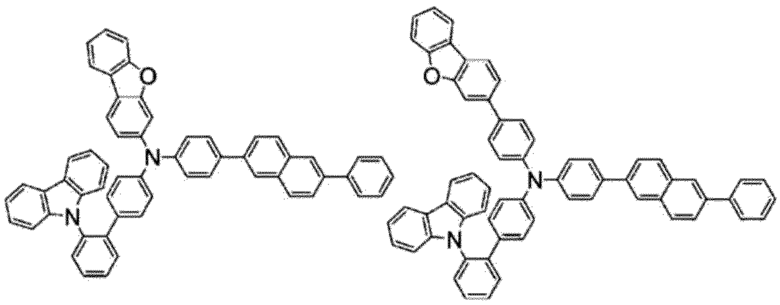

(8 0) (8 1)

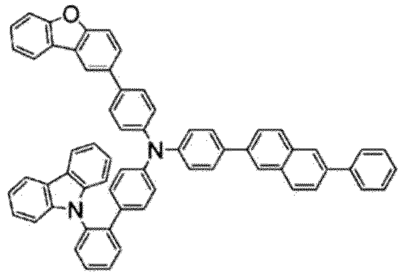

(8 2)

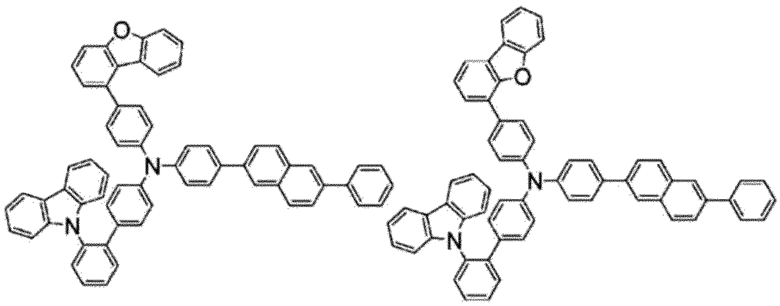

(8 3) (8 4)

# FIG. 8

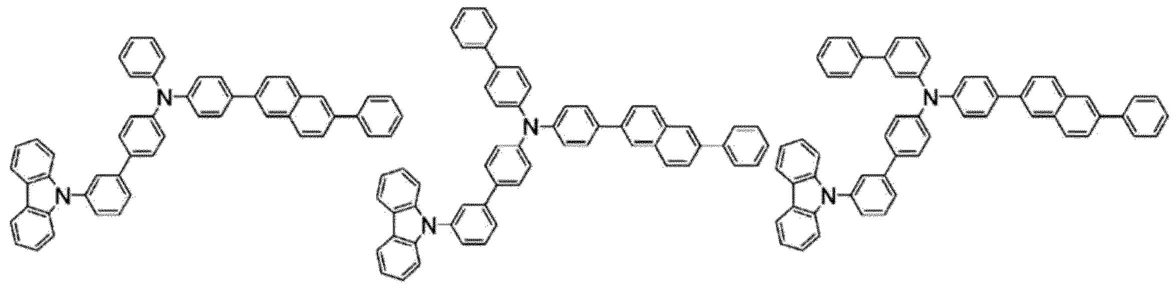

(85) (86) (87)

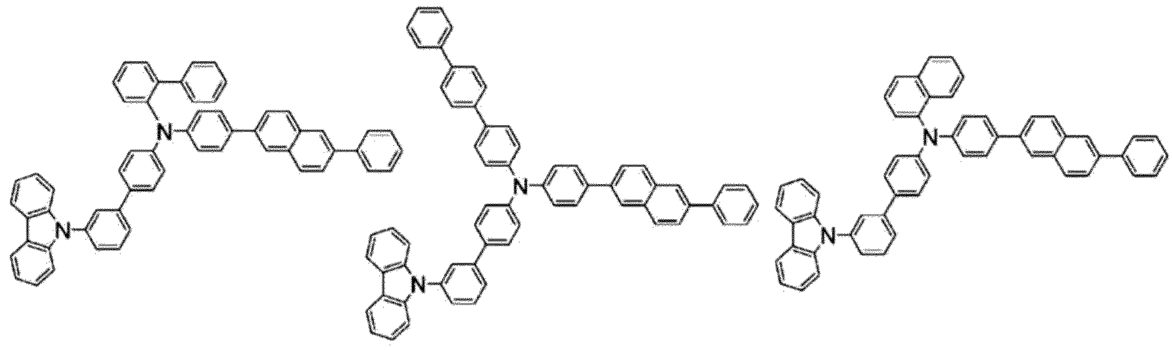

(88) (89) (90)

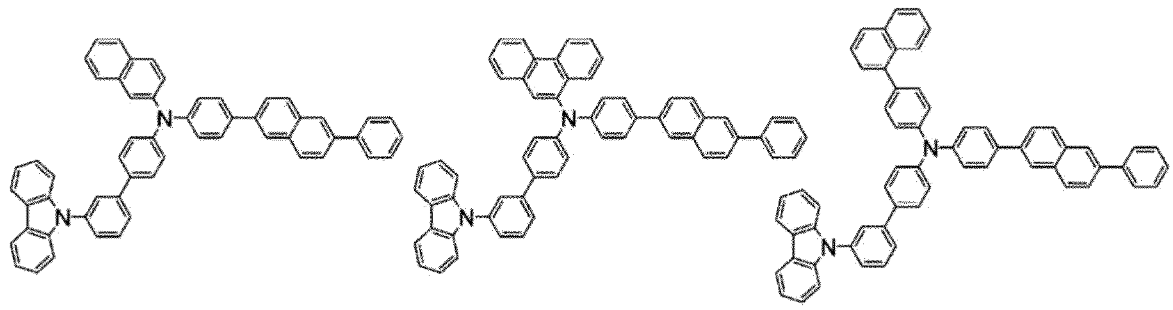

(91) (92) (93)

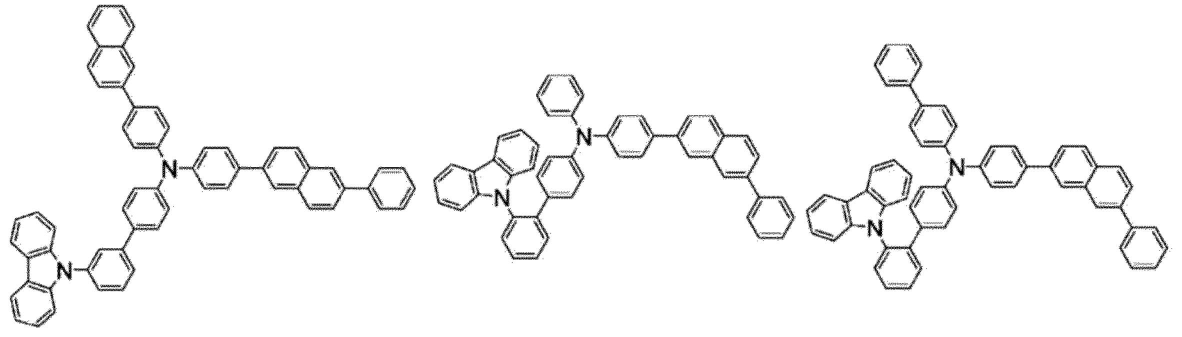

(94) (95) (96)

# FIG. 9

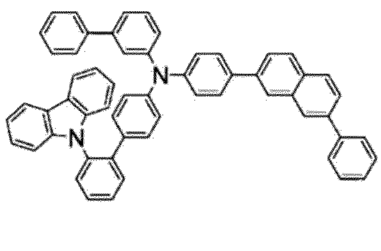

(97)

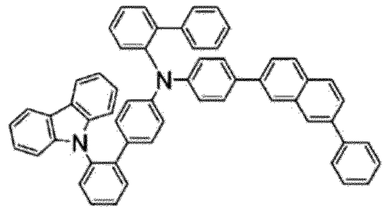

(98)

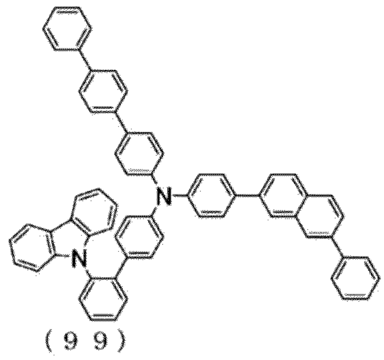

(99)

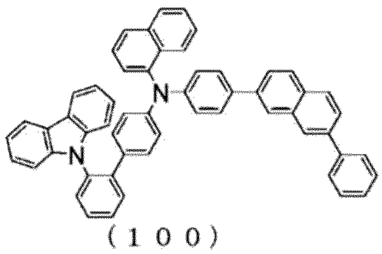

(100)

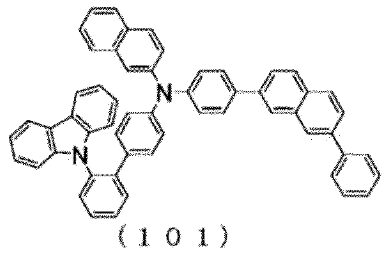

(101)

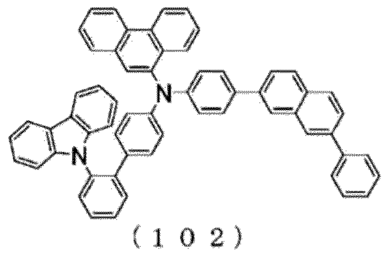

(102)

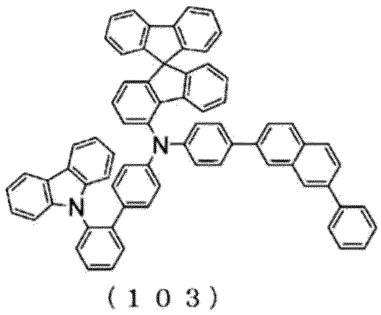

(103)

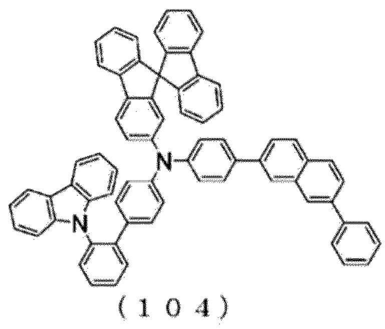

(104)

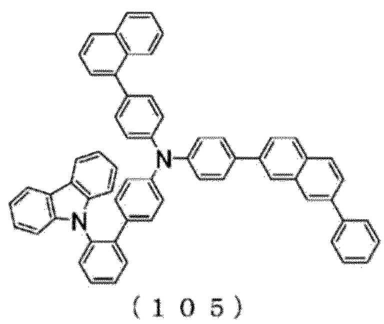

(105)

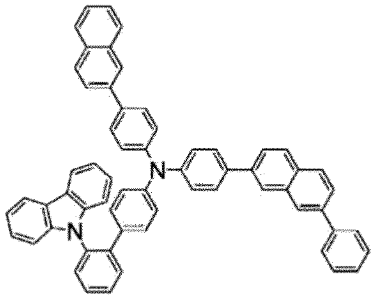

(106)

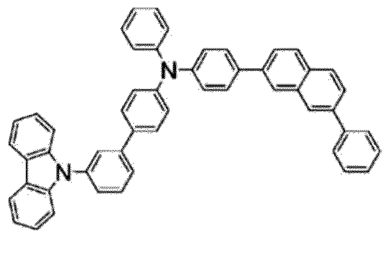

(107)

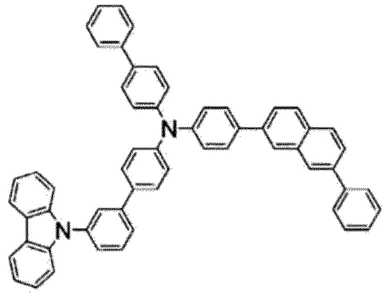

(108)

# FIG. 10

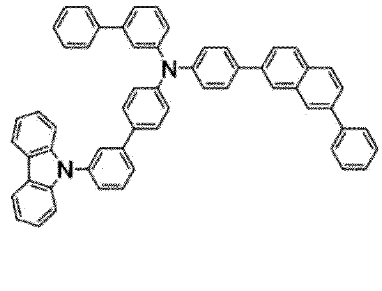

(109)

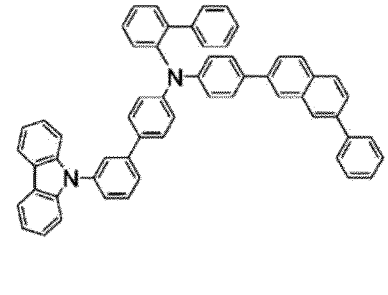

(110)

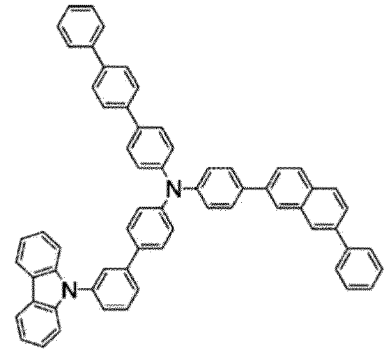

(111)

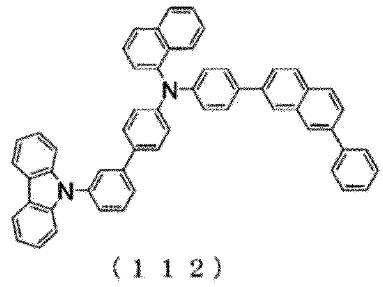

(112)

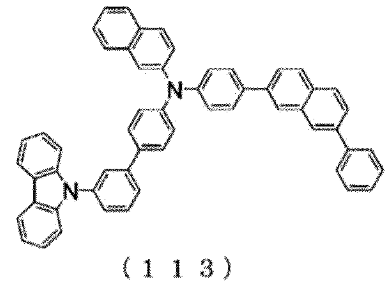

(113)

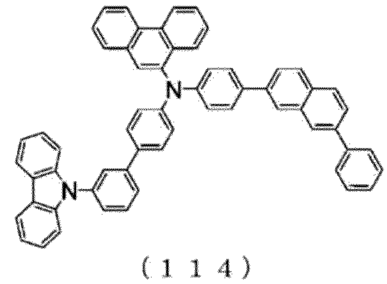

(114)

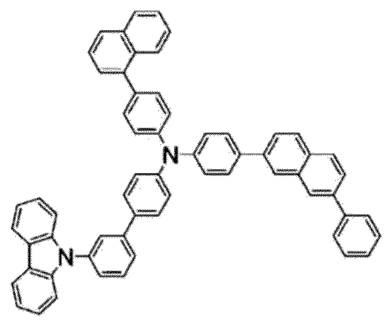

(115)

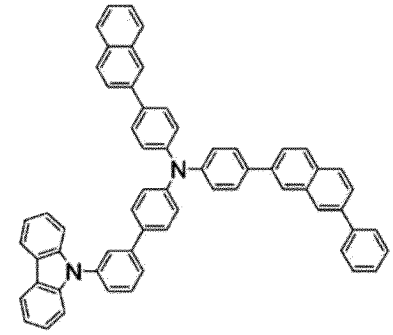

(116)

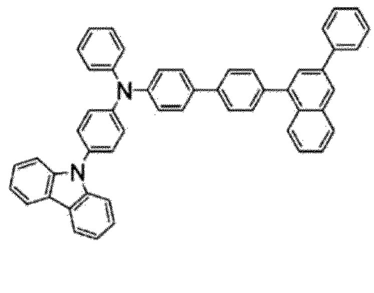

(117)

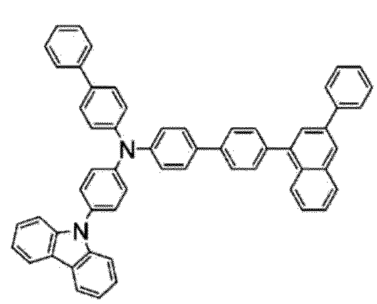

(118)

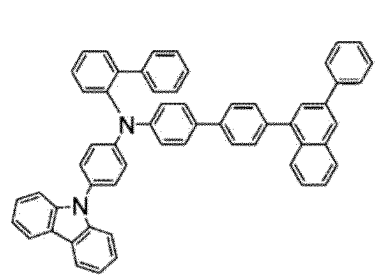

(119)

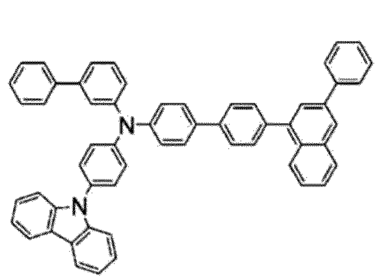

(120)

# FIG. 11

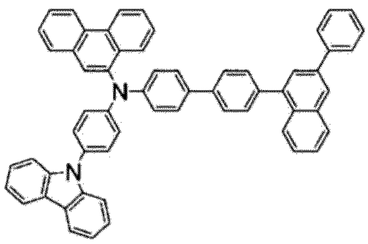

(121)

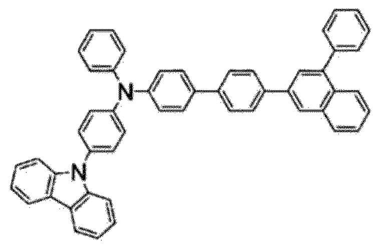

(122)

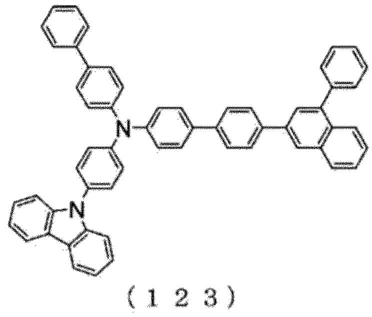

(123)

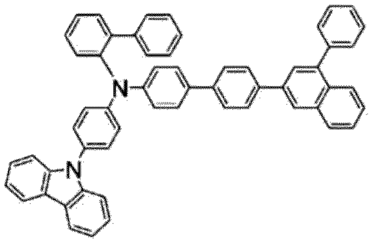

(124)

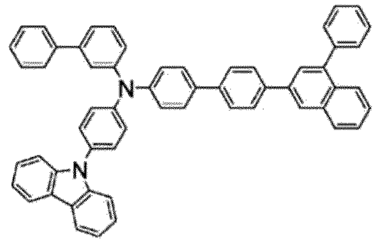

(125)

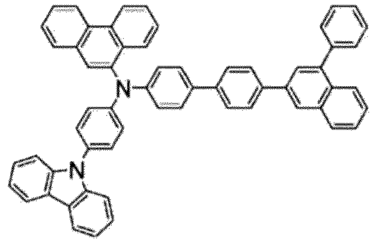

(126)

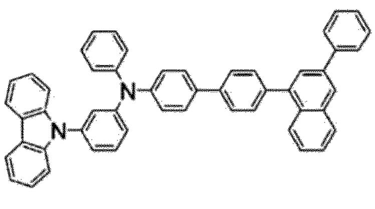

(127)

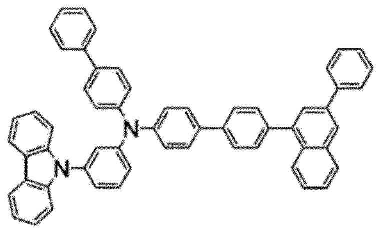

(128)

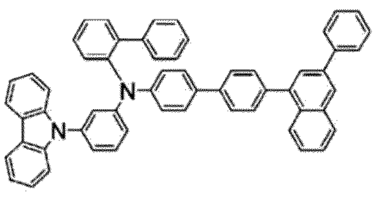

(129)

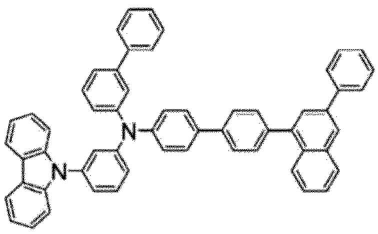

(130)

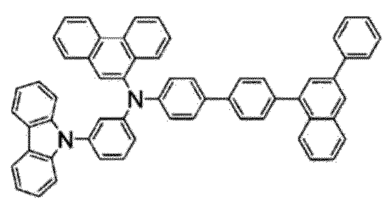

(131)

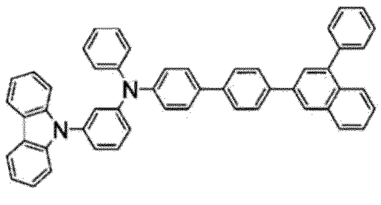

(132)

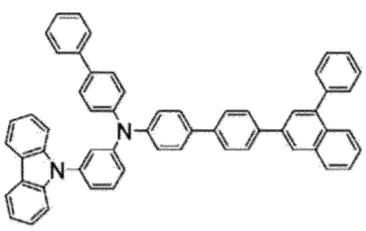

(133)

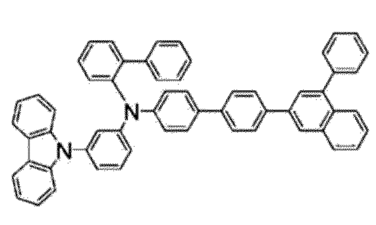

(134)

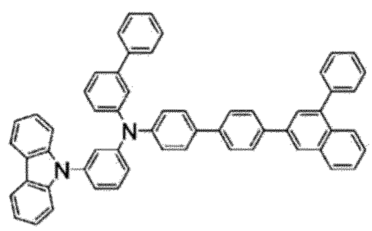

(135)

# FIG. 12

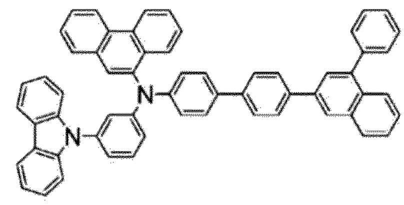

(136)

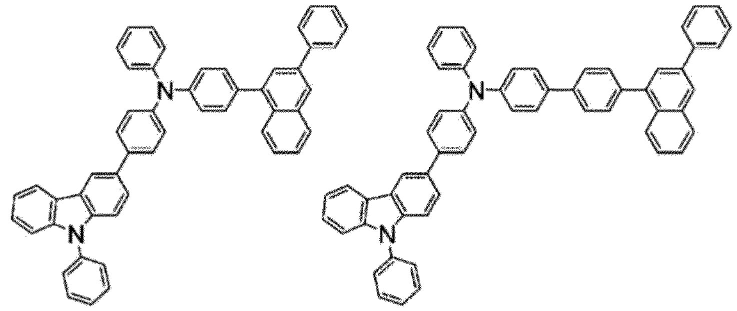

(137)

(138)

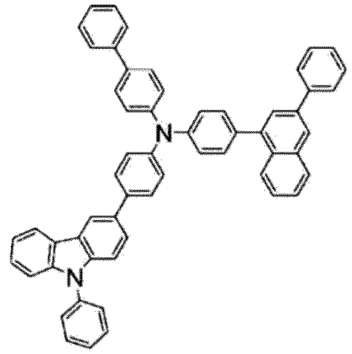

(139)

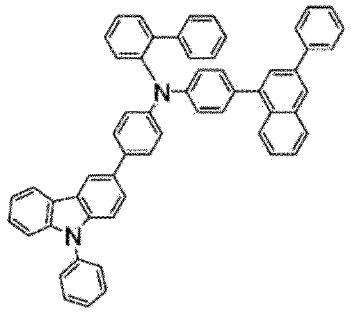

(140)

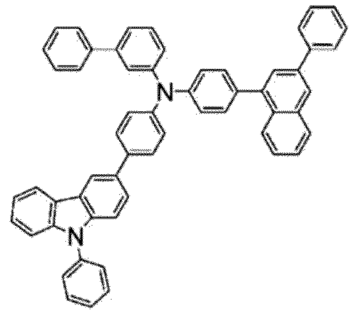

(141)

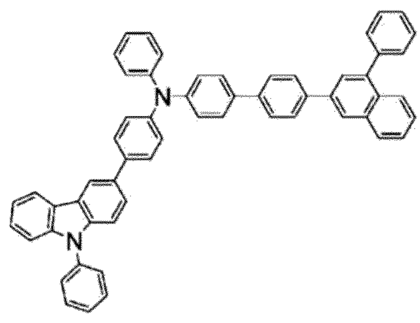

(142)

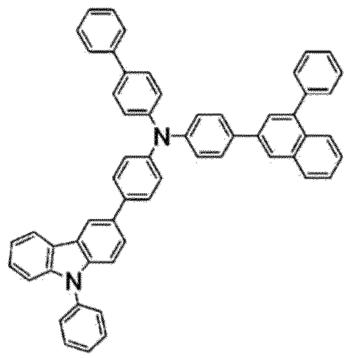

(143)

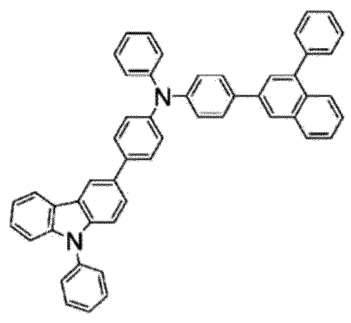

(144)

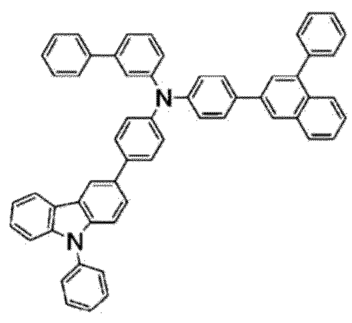

(145)

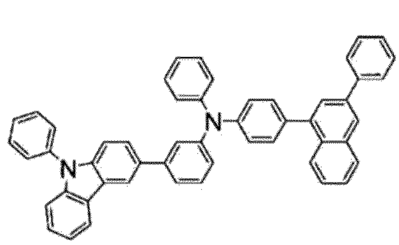

(146)

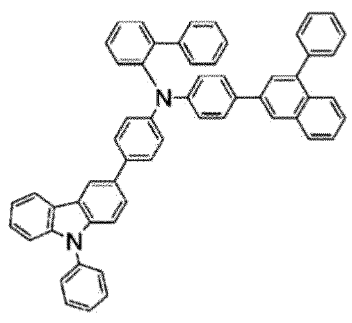

(147)

# FIG. 13

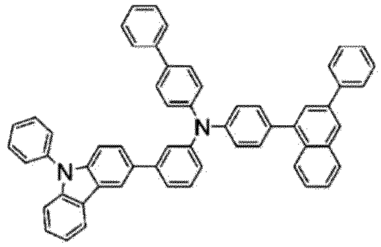

(148)

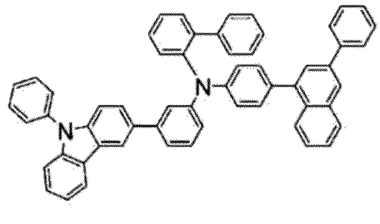

(149)

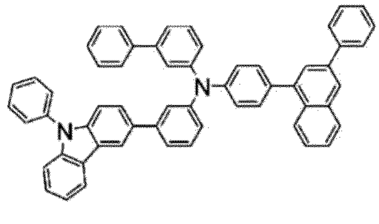

(150)

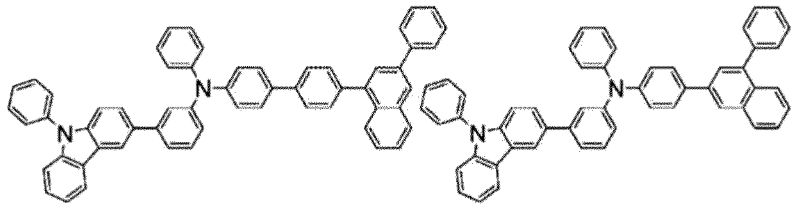

(151)

(152)

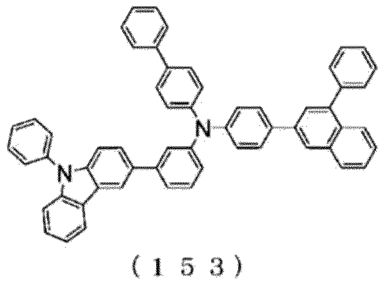

(153)

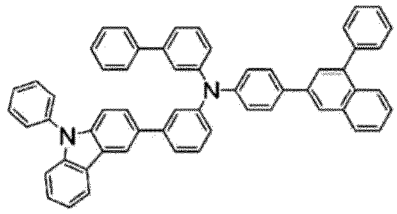

(154)

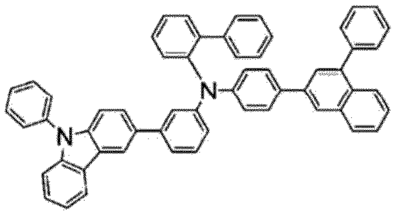

(155)

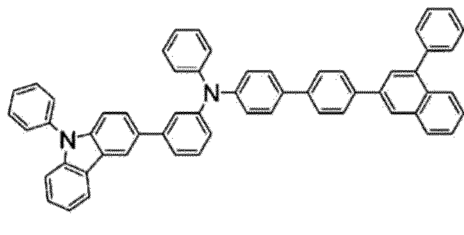

(156)

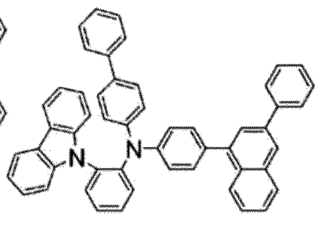

(157)

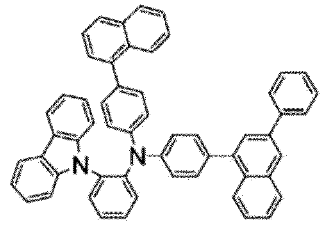

(158)

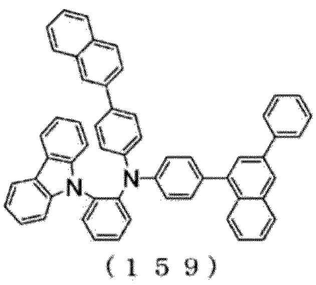

(159)

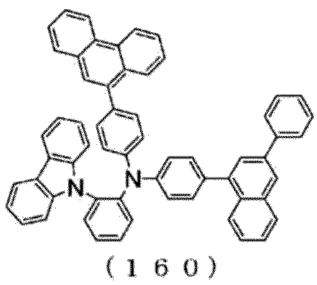

(160)

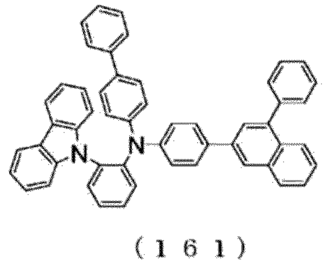

(161)

# FIG. 14

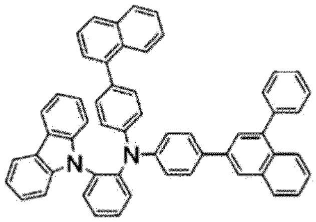
(162)

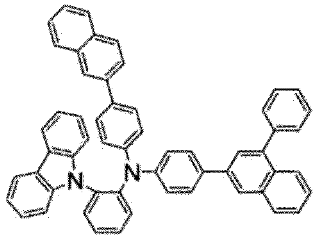
(163)

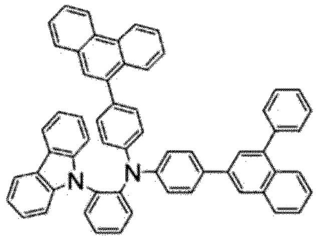
(164)

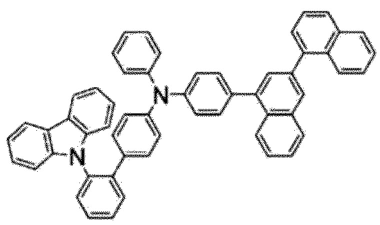
(165)

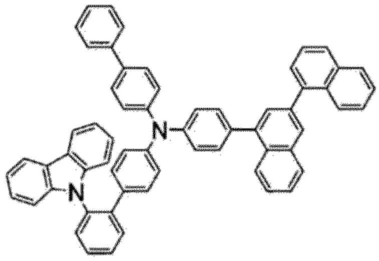
(166)

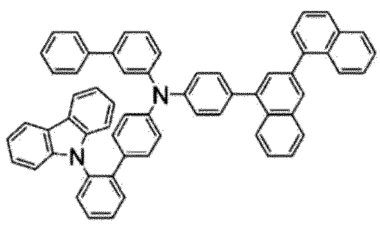
(167)

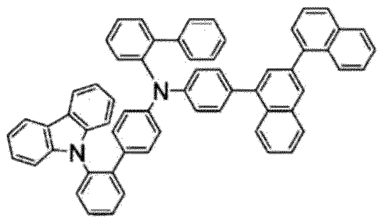
(168)

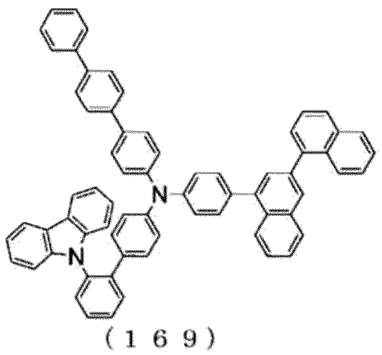
(169)

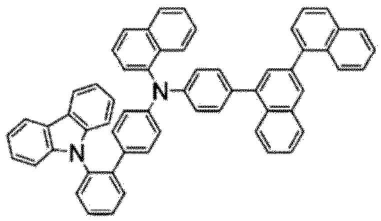
(170)

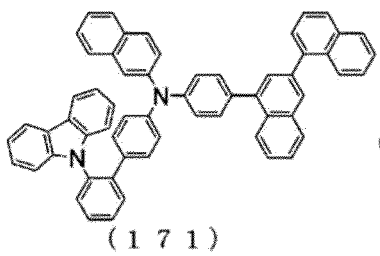
(171)

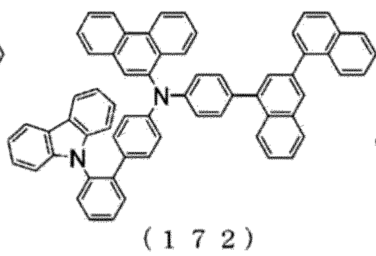
(172)

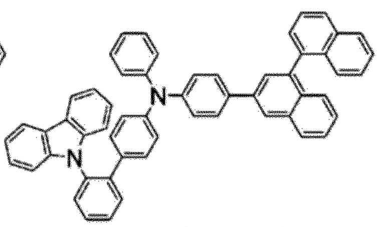
(173)

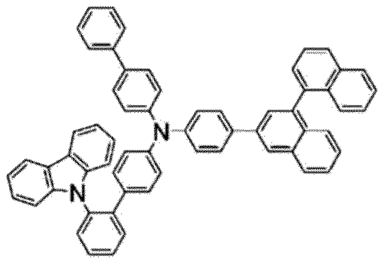
(174)

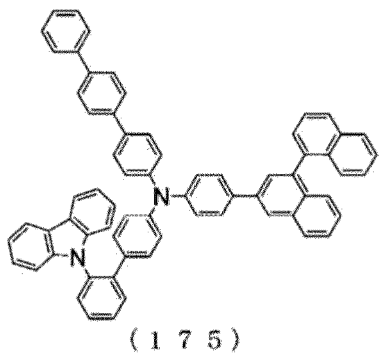
(175)

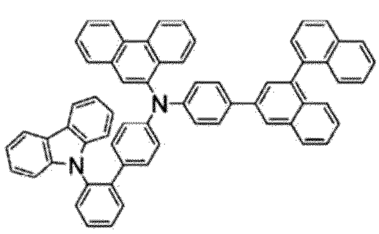
(176)

# FIG. 15

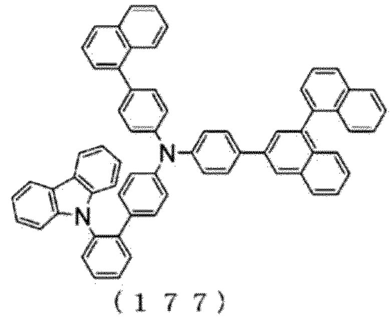
(177)

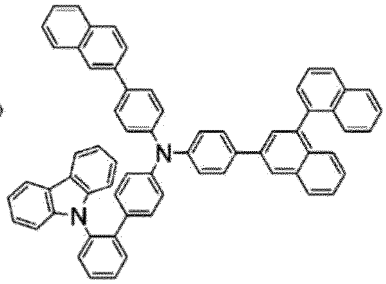
(178)

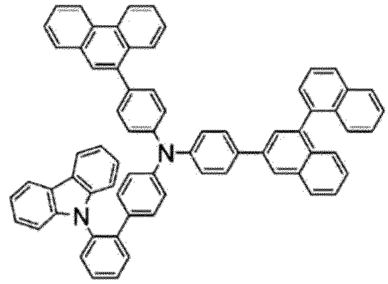
(179)

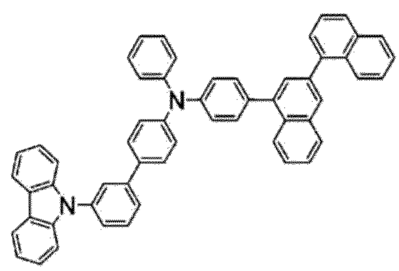
(180)

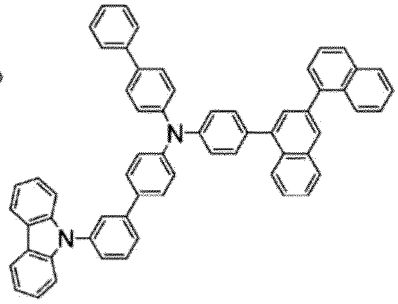
(181)

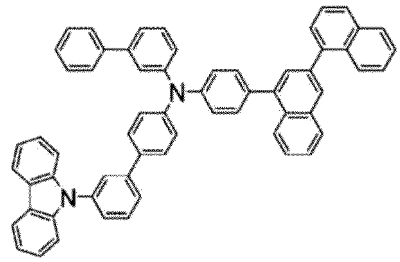
(182)

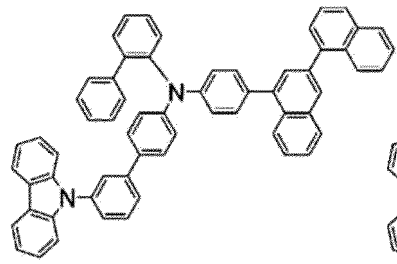
(183)

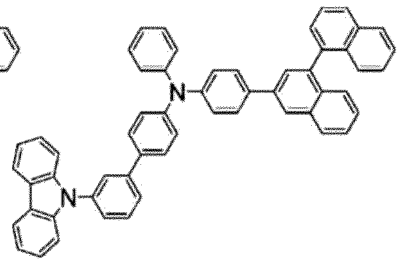
(184)

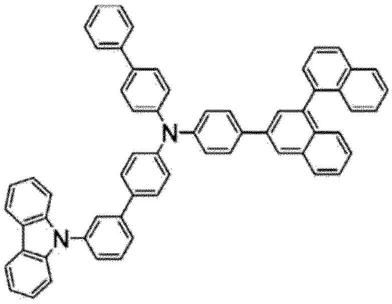
(185)

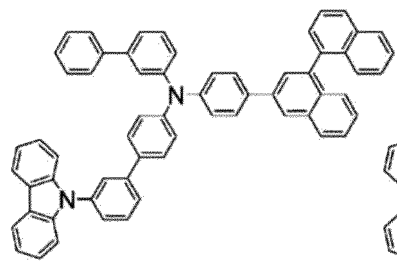
(186)

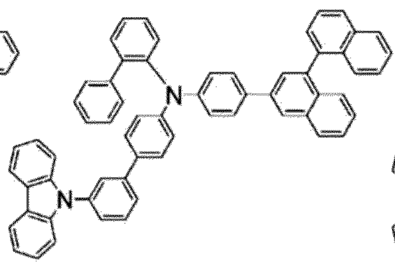
(187)

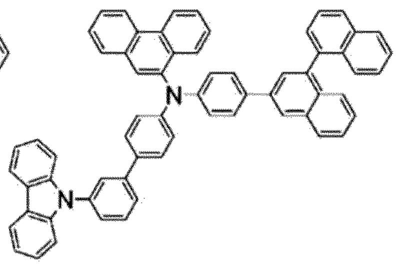
(188)

# FIG. 16

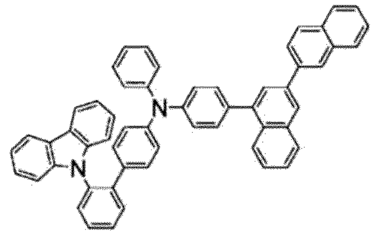

(189)

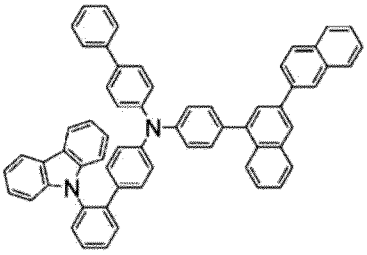

(190)

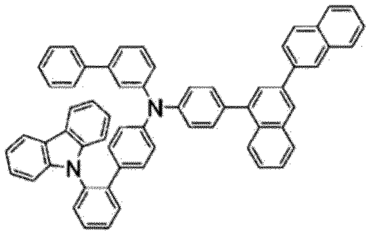

(191)

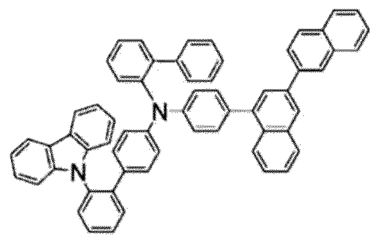

(192)

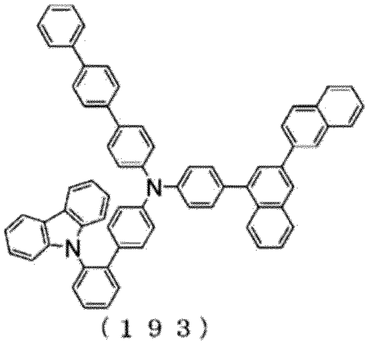

(193)

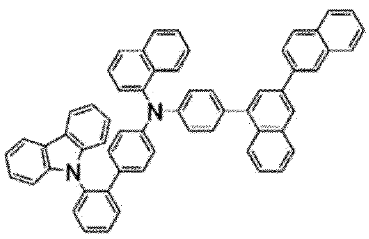

(194)

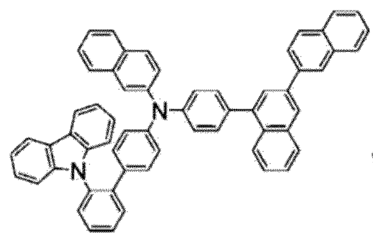

(195)

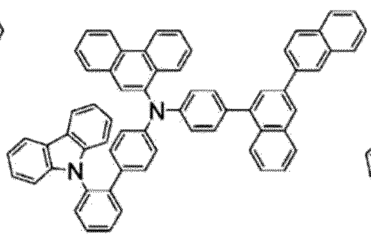

(196)

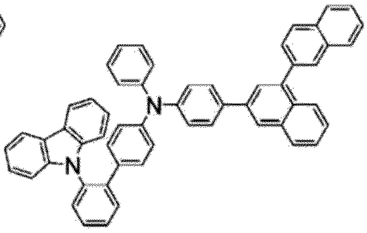

(197)

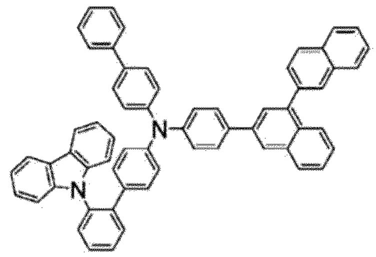

(198)

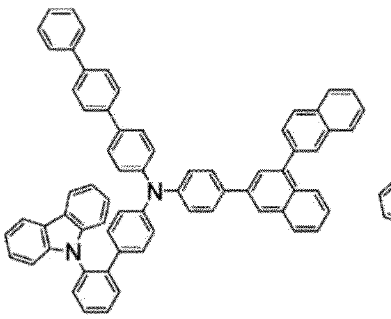

(199)

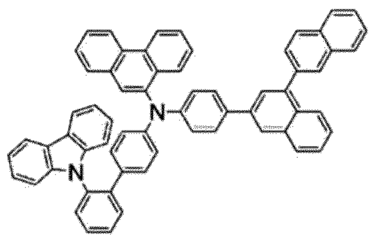

(200)

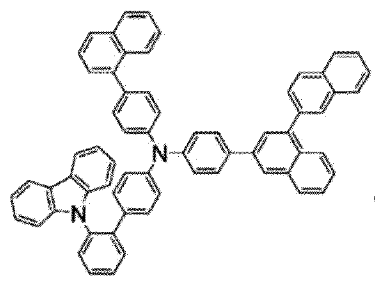

(201)

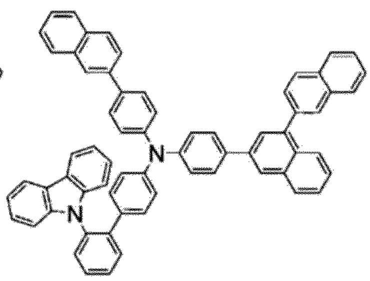

(202)

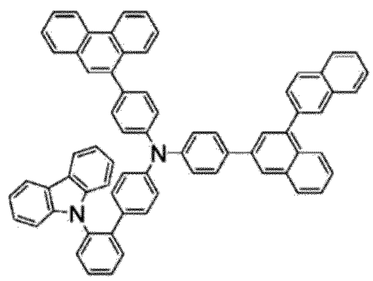

(203)

# FIG. 17

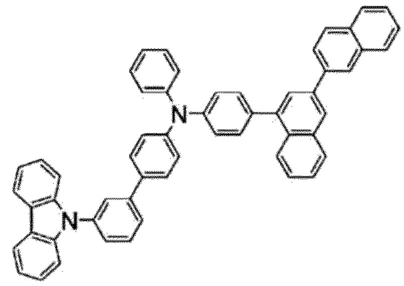

(2 0 4)

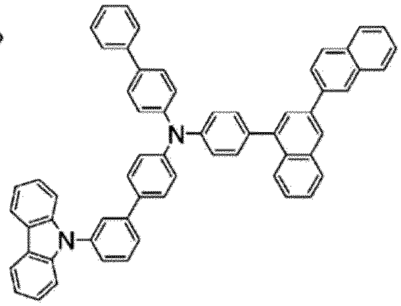

(2 0 5)

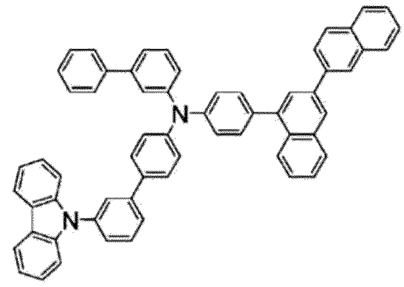

(2 0 6)

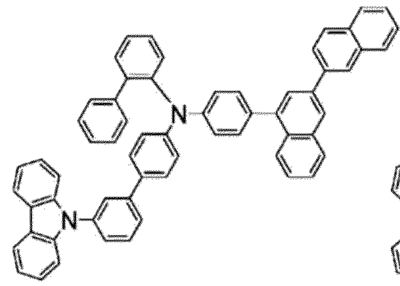

(2 0 7)

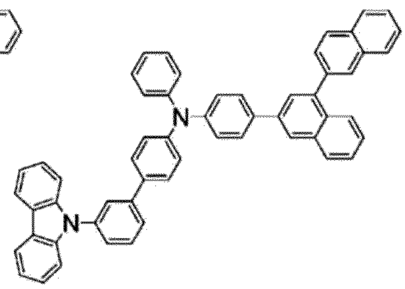

(2 0 8)

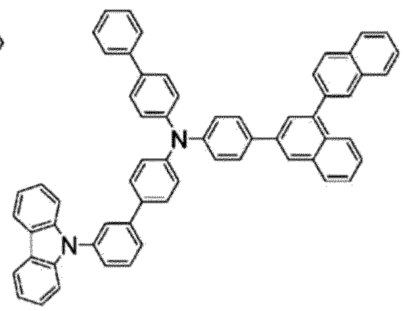

(2 0 9)

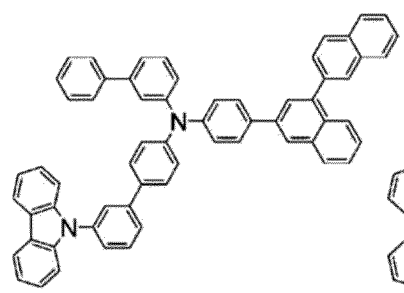

(2 1 0)

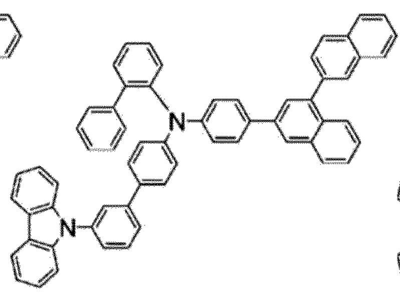

(2 1 1)

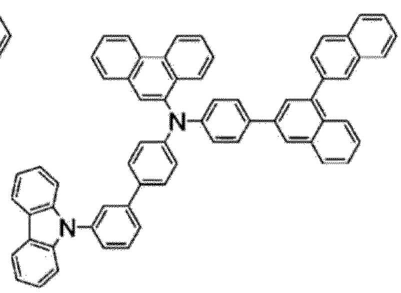

(2 1 2)

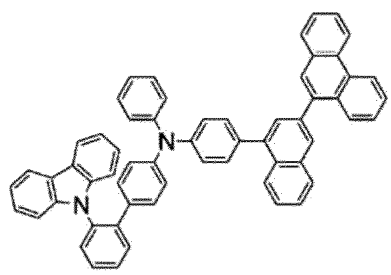

(2 1 3)

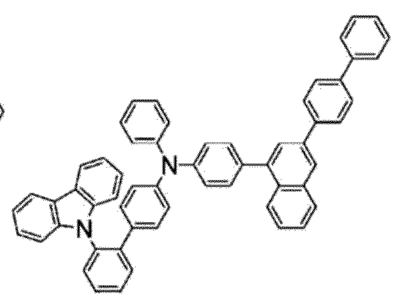

(2 1 4)

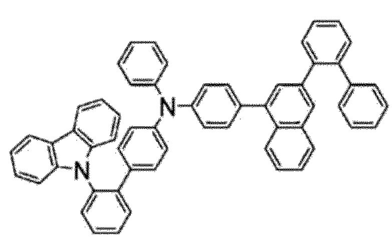

(2 1 5)

FIG. 18

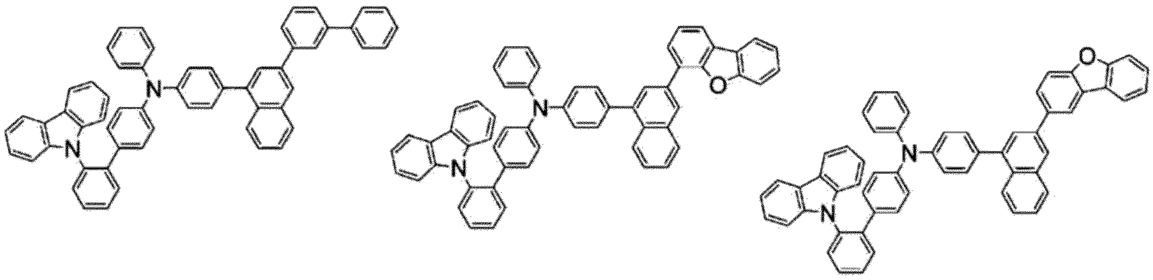

(216) (217) (218)

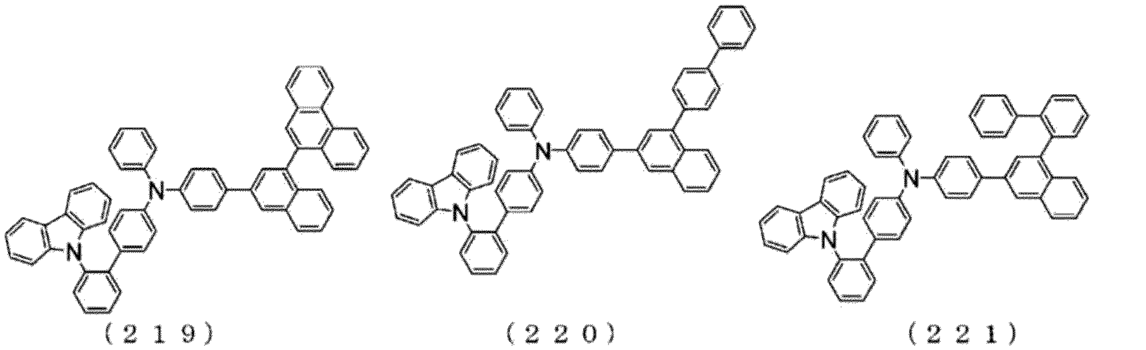

(219) (220) (221)

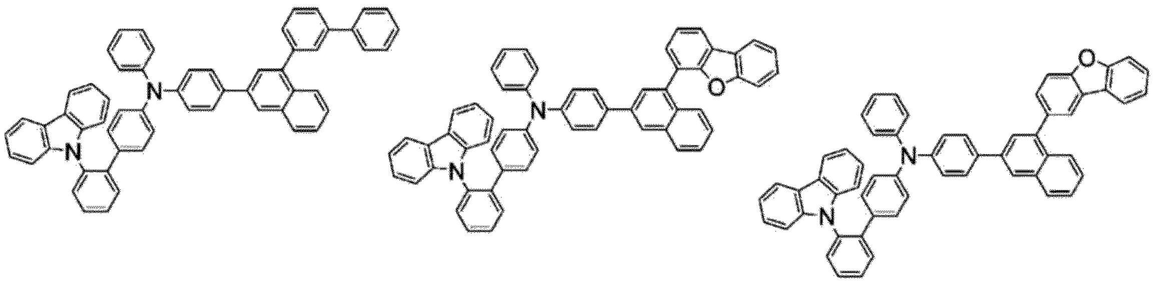

(222) (223) (224)

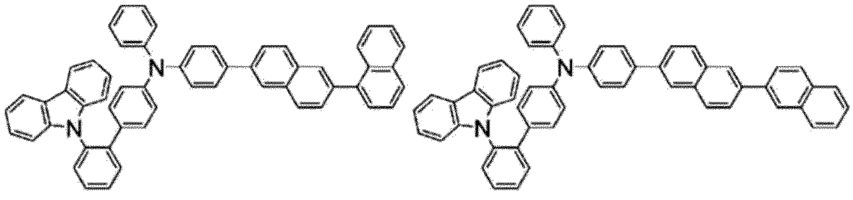

(225) (226)

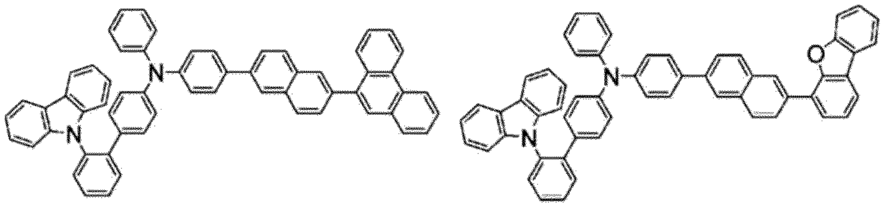

(227) (228)

FIG. 19

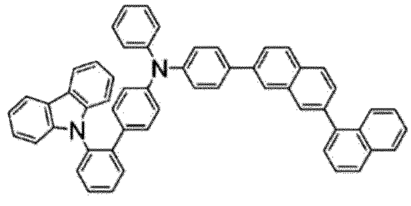

(229)

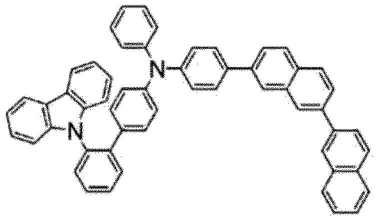

(230)

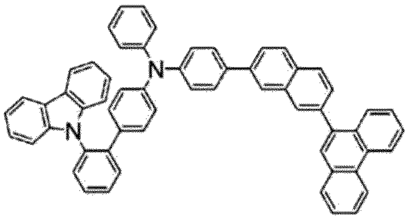

(231)

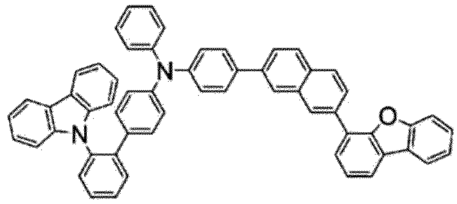

(232)

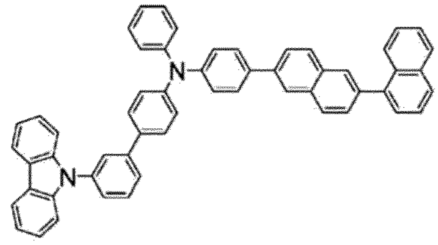

(233)

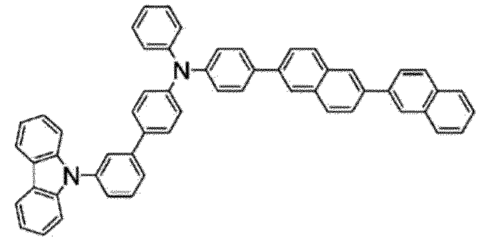

(234)

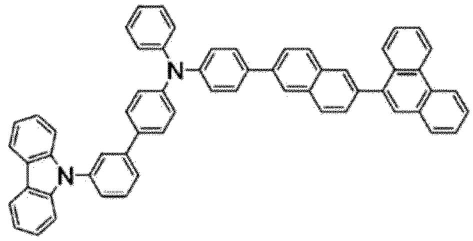

(235)

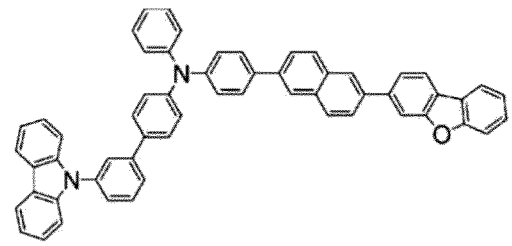

(236)

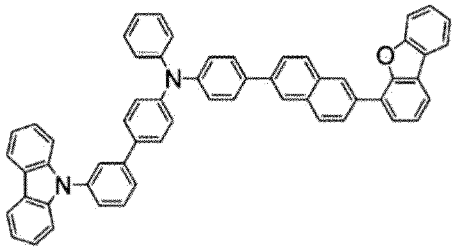

(237)

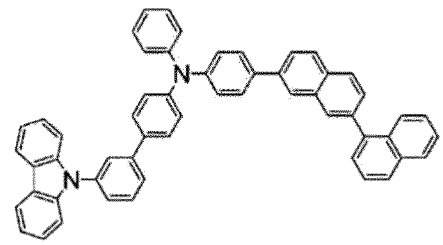

(238)

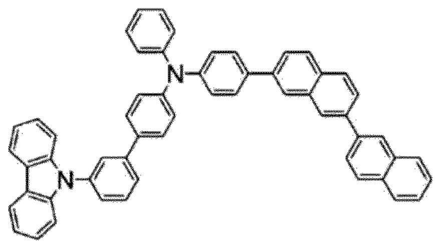

(239)

FIG. 20

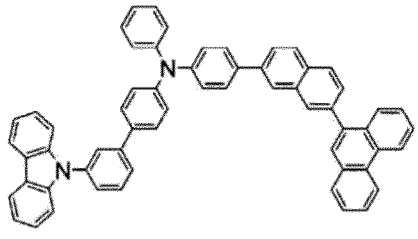

(240)

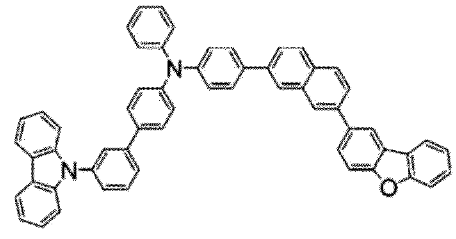

(241)

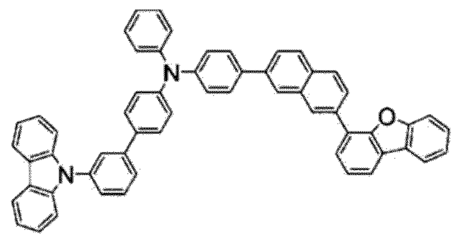

(242)

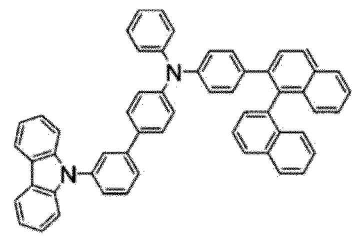

(243)

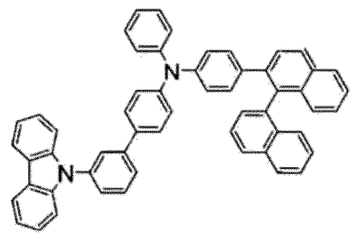

(244)

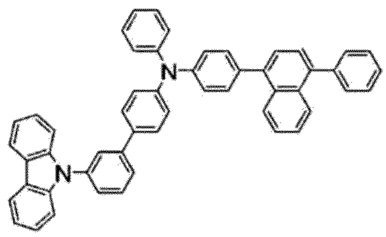

(245)

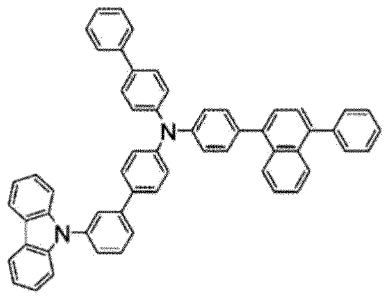

(246)

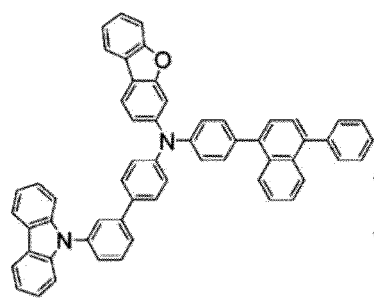

(247)

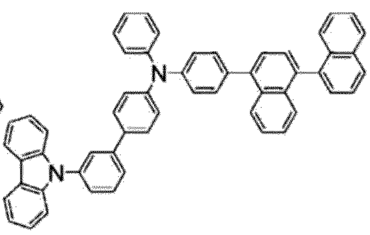

(248)

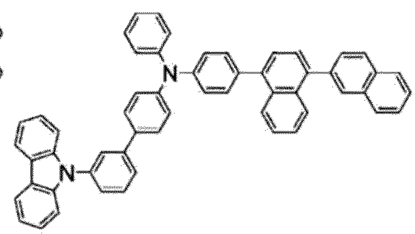

(249)

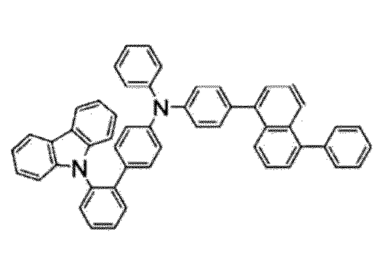

(250)

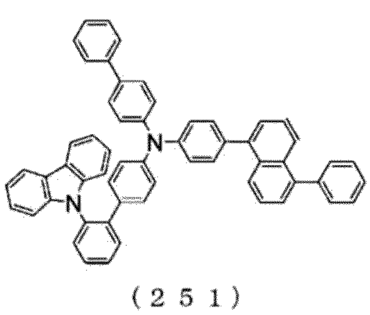

(251)

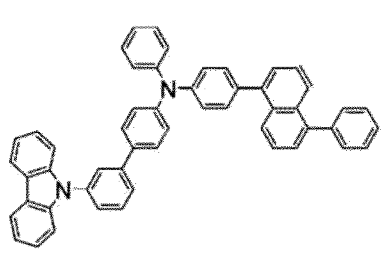

(252)

# FIG. 21

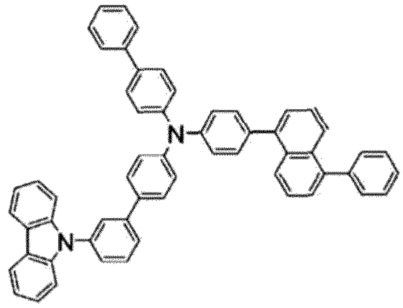

(253)

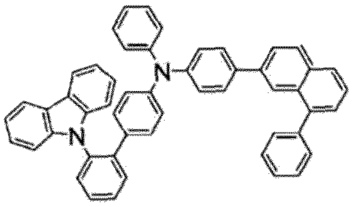

(254)

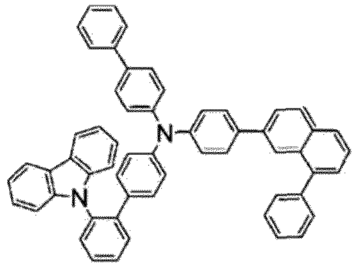

(255)

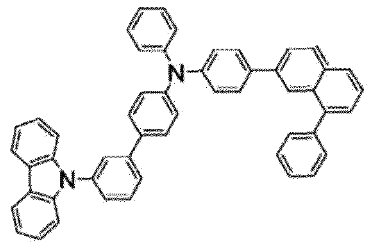

(256)

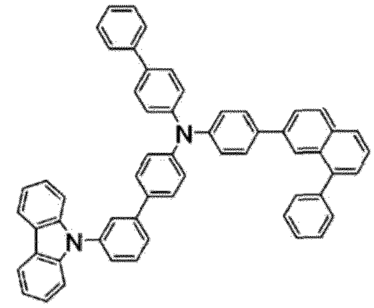

(257)

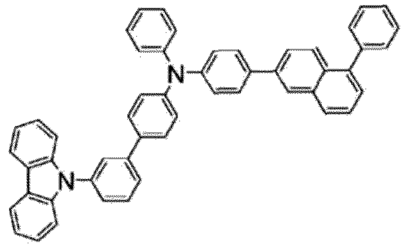

(258)

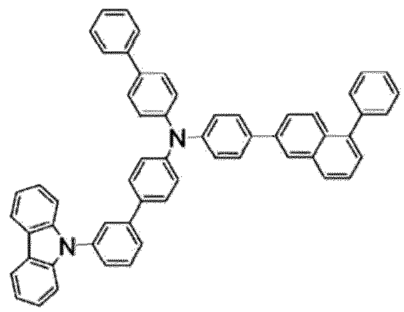

(259)

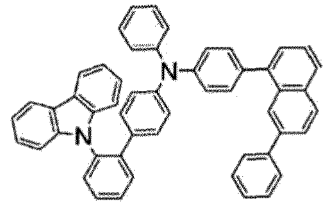

(260)

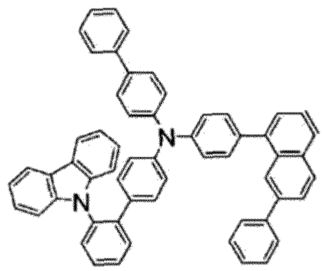

(261)

# FIG. 22

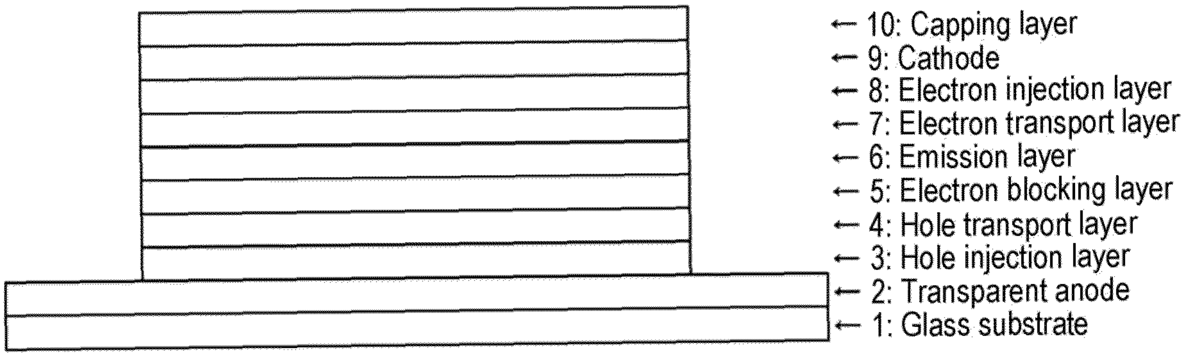

**INTERNATIONAL SEARCH REPORT**

International application No.
**PCT/IB2023/058126**

**A. CLASSIFICATION OF SUBJECT MATTER**

***C07D 209/86***(2006.01)i; ***C07D 307/91***(2006.01)i; ***C09K 11/06***(2006.01)i; ***H05B 33/14***(2006.01)i; ***H10K 50/10***(2023.01)i
FI: C07D209/86 CSP; C07D307/91; C09K11/06 630; C09K11/06 645; H05B33/14; H10K50/10

According to International Patent Classification (IPC) or to both national classification and IPC

**B. FIELDS SEARCHED**

Minimum documentation searched (classification system followed by classification symbols)

C07D209/86; C07D307/91; C09K11/06; H05B33/14; H10K50/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2022/154283 A1 (LG CHEM, LTD.) 21 July 2022 (2022-07-21) claims, in particular, example 1, p. 50, line 5, 1st and 4th compounds from the left, p. 53, line 4, 1st compound from the left | 1-12 |
| X | KR 10-2022-0049965 A (LG CHEM, LTD.) 22 April 2022 (2022-04-22) claims, in particular, p. 8, line 4, 3 compounds, p. 9, line 4, 3 compounds | 1-12 |
| X | WO 2022/080927 A1 (LG CHEM, LTD.) 21 April 2022 (2022-04-21) claims, in particular, p. 54, line 3, 4 compounds, p. 56, line 3, 4 compounds | 1-12 |
| X | WO 2022/010305 A1 (DUK SAN NEOLUX CO., LTD.) 13 January 2022 (2022-01-13) claims, in particular, compounds, pp. 9, 11, 15, 54-55, 61, 77 | 1-12 |
| X | CN 113683603 A (SHAANXI LIGHTE OPTOELECTRONICS MATERIAL CO., LTD.) 23 November 2021 (2021-11-23) claims, in particular, compounds 1, 13, 15, 22, 44, 54-72, 75, 197, 200-201, 203 | 1-12 |
| X | KR 10-2021-0122168 A (LG CHEM, LTD.) 08 October 2021 (2021-10-08) claims, in particular, compounds 70-71, 85-86, 99 | 1-12 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 October 2023** | **10 October 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No. **PCT/IB2023/058126**

**C. DOCUMENTS CONSIDERED TO BE RELEVANT**

| Category* | Citation of document, with indication, where appropriate, of the relevant passages | Relevant to claim No. |
|---|---|---|
| X | JP 2019-135228 A (SAMSUNG DISPLAY CO., LTD.) 15 August 2019 (2019-08-15) claims, in particular, compounds A16-A17, B16-B17, C16-C17, D16-D17, E16-E17, F16-F17, G16-G17 | 1-12 |

Form PCT/ISA/210 (second sheet) (January 2015)

**INTERNATIONAL SEARCH REPORT**
**Information on patent family members**

International application No.
**PCT/IB2023/058126**

| Patent document cited in search report | Publication date (day/month/year) | Patent family member(s) | Publication date (day/month/year) |
|---|---|---|---|
| WO 2022/154283 A1 | 21 July 2022 | KR 10-2022-0102317 A | |
| KR 10-2022-0049965 A | 22 April 2022 | (Family: none) | |
| WO 2022/080927 A1 | 21 April 2022 | CN 115916773 A | |
| | | KR 10-2022-0050806 A | |
| WO 2022/010305 A1 | 13 January 2022 | KR 10-2022-0007767 A | |
| | | CN 115884961 A | |
| CN 113683603 A | 23 November 2021 | WO 2022/199449 A1 | |
| KR 10-2021-0122168 A | 08 October 2021 | (Family: none) | |
| JP 2019-135228 A | 15 August 2019 | US 2020/0235297 A1 claims, in particular, compounds A16-A17, B16-B17, C16-C17, D16-D17, E16-E17, F16-F17, G16-G17 | |
| | | EP 3518303 A1 | |
| | | KR 10-2019-0091410 A | |
| | | CN 110078631 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5792557 A
- US 5639914 A
- US 7759030 B
- JP 2009076817 A
- JP 6674892 B
- JP 2684932 B
- KR 101888249 B1
- KR 102259465 B1
- KR 102078171 B1


### Non-patent literature cited in the description


- *Proceedings of the ninth Applied Physics Lecture Series*, 2001, 55-61
- *Proceedings of the ninth Applied Physics Lecture Series*, 2001, 23-31
- *Appl. Phys. Let.*, 2011, vol. 98, 083302
- *proceedings of the third meeting*, 2006, 13-14